# EUROPEAN PATENT SPECIFICATION

(11) **EP 0 412 006 B1**
(45) Date of publication and mention of the grant of the patent: **29.11.2000**
(21) Application number: 90402196.1
(22) Date of filing: 31.07.1990
(51) Int. Cl.: C12N 15/82, C12N 15/29, C12N 5/10, A01H 5/00

(54) **Plants with modified flowers, seeds or embryos**
Pflanzen mit modifizierten Blüten, Samen oder Embryos
Plantes à fleurs, semences ou embryons modifiés

(30) Priority: 04.08.1989 EP 89402224
(43) Date of publication of application: 06.02.1991
(73) Proprietor: Aventis CropScience N.V., 9000 Gent (BE)
(72) Inventor: De Greef, Willy, B-9000 Gent (BE); Van Emmelo, John, B-9110 Sint-Amandsberg (BE); De Oliveira, Dulce Eleonora, 20470 Lagoa, BR- Rio de Janeiro (BR); De Souza, Maria-Helena, B-9000 Gent (BE); Van Montagu, Marc, B-1050 Brussels (BE)
(74) Representative: Gutmann, Ernest

(56) References cited:
- EP-A- 0 198 288
- EP-A- 0 329 308
- EP-A- 0 343 947
- EP-A- 0 344 029
- JOURNAL OF CELLULAR BIOCHEMISTRY, Suppl., vol. 12C, 1988, UCLA Symposia on Molecular & Cellular Biology, Abstracts of the 17th Annual Meetings, The Molecular Basis of Plant Developement, 28 February - 10 April 1988, Alan R. Liss Inc., New York, NY (US); H.J. KLEE et al., p. 152, no. L 051
- THEOR. APPL. GENET., vol. 77, 1989, Springer-Verlag (DE); R. BERNATZKY et al., pp. 320-324
- THE PLANT CELL, vol. 1, January 1989, American Society of Plant Physiologists; C.S. GASSER et al., pp. 15-24
- JOURNAL OF CELLULAR BIOCHEMISTRY, suppl. 13D, 27 March - 07 April 1987, Alan R. Liss Inc., New York, NY (US); D. TWELL et al., p. 312, no. M 349
- THE PLANT CELL, vol. 1, April 1989, pages 403-413, American Society of Plant Physiologists; J.I. MEDFORD et al., pp. 403-413
- SCIENCE, vol. 244, 14 April 1989; P.N. BENFEY et al., pp. 174-181
- EMBO JOURNAL, vol. 7, no. 9, 1988, IRL Press Ltd., Oxford (GB); T. SCHMÜLLING et al., pp. 2621-2629
- MARIA HELENA DE SOUZA GOLDMAN: "Molecular genetic studies on stigmas and styles of Nicotiana Tabacum; Chapter 4: Cell ablation of the stigmatic secretory zone causes female sterility in tobacco.", 31. December 1999, UNIVERSITEIT GENT, GENT
- GOLDBERG, R.B. ET AL.: "Regulation of Gene expression during plant embryogenesis.", CELL, , 27. January 1989, vol. 56, no. , pages 149 to 160
- D'HALLUIN, K. ET AL.: "Engineering of herbicide-resistant alfalfa and evaluation under field conditions.", CROP SCIENCE, , 01. July 1990, vol. 30, no. 4, pages 866 to 871

## Description

This invention relates to a female-sterile plant and to its reproductive material (e.g., seeds), in which the cells have been transformed so that a foreign DNA sequence is stably integrated into their nuclear genome. The foreign DNA sequence of this invention contains a first foreign DNA (hereinafter the "female-sterility DNA") that: 1) encodes a first RNA, protein or polypeptide which, when produced or overproduced in a cell of a flower, particularly a female organ thereof, or a seed or an embryo of a plant, disturbs significantly the metabolism, functioning and/or development of the cell of the flower or seed or embryo; and 2) is in the same transcriptional unit as, and under the control of, a first promoter which is capable of directing expression of the female-sterility DNA selectively in cells of the flowers, particularly one or more of their female organs, or seeds or embryos of the plant. In particular, this invention relates to such a nuclear female-sterile plant and its reproductive material, in which the foreign DNA sequence of this invention is a foreign chimaeric DNA sequence that can also contain a second foreign DNA (the "marker DNA") that: encodes a second RNA, protein or polypeptide which, when present at least in a specific tissue or specific cells of the plant, renders the entire plant easily separable from other plants that do not contain the second RNA, protein or polypeptide at least in the specific tissue or specific cells; 2) is in the same transcriptional unit as, and under the control of, a second promoter which is capable of directing expression of the marker DNA in at least the specific tissue or the specific cells of the plant; and 3) is in the same genetic locus of the nuclear genome of the cells of the plant as the female-sterility DNA.

This invention also relates to a foreign chimaeric DNA sequence that contains at least one female-sterility DNA under the control of at least one first promoter and that can also contain, adjacent to the female-sterility DNA(s) and the first promoter(s), at least one marker DNA under the control of at least one second promoter.

This invention further relates to a vector that contains the foreign DNA sequence of this invention and is suitable for the transformation of a plant cell, whereby the foreign DNA sequence is stably integrated into the nuclear genome of the cell.

This invention still further relates to cells of a plant and to plant cell cultures, the nuclear genomes of which are transformed with the foreign DNA sequence.

This invention yet further relates to a process for producing a nuclear female-sterile male-fertile plant and its reproductive material containing the foreign DNA sequence in which the female-sterility DNA: 1) is under the control of the first promoter; 2) is stably integrated into the nuclear genome of the plant's cells; 3) can be expressed selectively in cells of each flower, particularly a female organ thereof, or each seed or each embryo of the plant in the form of the first RNA, protein or polypeptide; and optionally 4) is in the same genetic locus as the marker DNA under the control of the second promoter.

The invention further relates to a process for producing hybrid seeds, which grow into hybrid plants, by crossing: 1) the female-sterile plant of this invention which may include, in its nuclear genome, the marker DNA, preferably encoding a protein conferring a resistance to a herbicide on the plant; and 2) a female-fertile plant without the marker DNA in its genome. This invention particularly relates to such a process for producing hybrid seeds on a commercial scale, preferably in a substantially random population, without the need for extensive hand-labor.

### Background of the Invention

Hybridization of plants is recognized as an important process for producing offspring having a combination of the desirable traits of the parent plants. The resulting hybrid offspring often has the ability to outperform the parents in different traits, such as in yield, adaptability to environmental changes, and disease resistance. This ability is called "heterosis" or "hybrid vigor". As a result, hybridization has been used extensively for improving major crops, such as corn, sugar beet and sunflower. For a number of reasons, primarily related to the fact that most plants are capable of undergoing both self-pollination and cross-pollination, the controlled cross-pollination of plants without significant self-pollination, to produce a harvest of hybrid seeds, has been difficult to achieve on a commercial scale.

In nature, the vast majority of crop plants produce male and female reproductive organs on the same plant, usually in close proximity to one another in the same flower. This favors self-pollination. Some plants, however, are exceptions as a result of the particular morphology of their reproductive organs which favors cross-pollination. These plants produce hybrid offspring with improved vigor and adaptability. One such morphology in Cannabis ssp. (hemp) involves male and female reproductive organs on separate plants. Another such morphology in Zea mays (corn) involves male and female reproductive organs on different parts of the same plant. Another such morphology in Elaeis guineensis (oil palm) involves male and fertile female gametes which become fertile at different times in the plant's development.

Some other plant species, such as Ananas comosus (pineapple), favor cross-pollination through the particular physiology of their reproductive organs. Such plants have developed a so-called "self-incompatibility system" whereby the pollen of one plant is not able to fertilize the female gamete of the same plant or of another plant with the same genotype.

Some other plant species favor cross-pollination by naturally displaying the so-called genomic characteristic of "male sterility". By this characteristic, the plants' anthers degenerate before pollen, produced by the anthers, reaches maturity. See: "Male-sterility in Higher Plants", M.L.H. Kaul, 1987, in: Monographs on Theoretical and Applied Genetics 10, Edit. Springer Verlag. Such a natural male-sterility characteristic is believed to result from a wide range of natural mutations, most often involving recessive deficiencies, and this characteristic can not easily be maintained in plant species that predominantly self-pollinate, since under natural conditions, no seeds will be produced.

Some other plants favor cross-pollination by naturally displaying the character of "female-sterility" due to a deficient functioning of either the female gametophyte, the female gamete, the female zygote, or the seed. These plants produce no viable seeds. There are many different mutations that can lead to this condition, involving all stages of development of a specific tissue of the female reproductive organ. This characteristic distinguishes female-sterility from the more widely known phenomena of male-sterility and self-incompatibility. Although reducing the number of offspring a species can produce, the female-sterility trait has some evolutionary advantages in nature for some plants, especially for perennials. In perennials, the rate of vegetative growth is to a large extent determined by the distribution of biomass between vegetative and reproductive plant tissues. Female-sterile plants therefore tend to grow more vigorously than the female-fertile plants.

Although female-sterility inducing mutations probably occur as frequently as male-sterility inducing mutations, female-sterility inducing mutations are much less used in plant breeding and seed production and consequently much less studied, and only few examples of such mutations exist.

A well documented illustration of natural female-sterility is the oil palm (Elaeis guineensis) where the so-called "pisifera" condition is characterized by the inability of the developing seed to produce a shell. As a result, the developing seed aborts in an early stage, and no ripe fruit is formed. The gene encoding the "pisifera" genotype acts as a semi-dominant allele. Plants homozygous for the allele are not capable of producing a seed shell and consequently no ripe fruit or seeds. Plants heterozygotic for the allele produce ripe fruit and seeds with a thin shell (0.5 to 2 mm), while wild-type plants (which do not carry the allele) produce ripe fruit and seeds with shells of 2 to 6 mm thickness. These two genotypes are indistinguishable in seed yield, and their genotype is determined by that of the female parent plant. In oil palm breeding, the "pisifera" type is used as the male parent plant in all commercial seed production. By crossing pollen from the "pisifera" palms with the wild-type female parent plants, a homogeneous F1 hybrid seed population, producing thin-shelled fruit, is obtained.

Another example of a plant with a natural female-sterility, used for the commercial production of hybrid seed, is alfalfa. Alfalfa was known to have male-sterility genes, but in testing a hybrid seed production system in which male-sterile and male fertile plants were sown in separate bands, it appeared that a negligeable amount of hybrid seeds was produced. This low production was due to the fact that honeybees, responsible for pollination, have low affinity for male-sterile plants, favoring the self-pollination of the male-fertile plants. To obtain good seed set, it seemed necessary to interplant very closely to each other (thus not in separate rows) the male-fertile and the male-sterile parent plants. This was made possible when a female-sterility gene was discovered and bred into the male-fertile plants. Consequently, the only seeds which could be produced in the randomly sown plots were hybrid seeds obtained by cross-pollination between the female-sterile and the male-sterile parent plants.

For other crops, female-sterility has been reported, such as sorghum (Casady et al (1960) J. Hered. 51, 35-38), cotton (Tustus and Meyer (1963) J. Hered. 54, 167-168), tomato (Honma and Pratak (1964) J. Hered. 55, 143-145), wheat (Gotzov and Dzelepov (1974) Gen. Plant Breed. 7, 480-487), and pearl millet (Hanna and Powel (1974) J. Hered. 65, 247-249). There are, however, several problems in maintaining the female-sterile lines, and for this reason, such lines are not used on a commercially important scale.

Compared with male-sterility, the use of female-sterility offers some other advantages in the production of hybrid seeds. Female-sterility allows the production of fruits without seeds and enhanced vegetative biomass production and can, in some cases, induce more flower-setting within one season.

### Summary of the Invention

In accordance with this invention a DNA, such as a nuclear DNA of a cell of a plant, is provided which contains a first chimeric DNA which comprises:
(a) a female sterility DNA encoding a first RNA, protein or polypeptide, capable, when produced in cells of a female reproductive organ of the plant, of disturbing significantly the metabolism, functioning and/or development of the female reproductive organ; and
(b) a first promoter capable of directing expression of this DNA. selectively in cells of the female reproductive organs of the plant, the female sterility DNA being in the same transcriptional unit as, and under the control of the first promoter.
The first promoter preferably directs expression especially in style cells, stigma cells, ovary cells, ovule cells and/or septum cells, particularly in stigma and/or style cells. The female-sterility DNA preferably encodes a ribonuclease, such as barnase.

The DNA of this invention can also contain a second chimeric DNA comprising:
(c) a marker DNA encoding a second RNA, protein or polypeptide which, when present at least in a specific tissue or in at least specific cells of a plant, renders the plant easily separable from other plants which do not contain the second RNA, protein or polypeptide in the specific tissue or specific cells; and
(d) a second promoter capable of directing expression of the marker DNA at least in the specific tissue or specific cells; the marker DNA being in the same transcriptional unit as, and under the control of, the second promoter.
The marker DNA is preferably a herbicide resistance gene.

Also in accordance with this invention is provided a DNA comprising the female-sterility DNA under the control of a first promoter, and that may also comprise the marker DNA and the second promoter, as well as at least one additional DNA encoding: a transit peptide capable of transporting the first protein or polypeptide and/or the second protein or polypeptide into a chloroplast or mitochondrion of a plant cell; and/or a secretory signal peptide capable of secreting the first protein or polypeptide, and/or the second protein or polypeptide out of a plant cell or plant tissue.

Further in accordance with this invention are provided; a cell of a plant, a plant cell culture, a plant, a plant seed, and a seedless fruit containing the DNA of this invention. Also provided are a pair of parent plants comprising a) a female-sterile parent plant containing the DNA of this invention, and b) a male-fertile parent plant.

In particular this invention provides a female-sterile plant containing a foreign DNA incorporated in the nuclear genome of its cells, wherein the foreign DNA comprises :
(a) a female-sterility DNA encoding a first RNA, protein or polypeptide, capable when produced in cells of a female reproductive organ of the plant, of disturbing significantly the metabolism, functioning and/or development of the female reproductive organ; and
(b) a first promoter capable of directing expression of this DNA selective in cells of the female reproductive organs of the plant, the female sterility DNA being in the same transcriptional unit as, and under the control of the first promoter,
provided that, if the first promoter is a promoter capable of directing expression of the female-sterility DNA selectively in female gametes, or in cells derived from female gametes, the nuclear genome of the plant is homozygous.

Still further in accordance with this invention a proces is provided for producing a female-sterile plant and reproductive material, e.g. seeds, thereof, by transforming a cell of a plant with a DNA comprising the female-sterility DNA under control of the first promoter, regenerating the female-sterile plant, and optionally, obtaining the reproduction material or progeny of the female-sterile plant.

Still further in accordance with this invention a process for producing a seed is provided which comprises the steps of:
(a) providing a female-sterile plant which contains a DNA comprising a female-sterility DNA under the control of a first promoter and a marker DNA, which is a gene conferring resistance to a herbicide, under the control of a second promoter,
(b) cross-pollinating i) the female-sterile plant, and ii) a female-fertile plant, preferably a male-sterile plant, without the marker DNA and/or the second promoter, and
(c) recovering the seed from the female-fertile plant,
wherein the process comprises removing undesired female-fertile plants by application of the herbicide.

### Detailed Description of the Invention

In accordance with this invention, a female-sterile male-fertile plant is produced from a single cell of a plant by transforming the plant cell in a well known manner to stably insert, into the nuclear genome of the cell, the foreign DNA sequence of this invention. The foreign DNA sequence comprises at least one female-sterility DNA that is under the control of, and fused at its 5' end to, the first promoter and is fused at its 3' end to suitable transcription termination (or regulation) signals, including a polyadenylation signal. Thereby, the first RNA, protein or polypeptide is produced or overproduced selectively in cells of all the flowers, particularly in one or more female organs thereof, and/or in all the seeds and/or in all the embryos of the plant so as to render the plant female-sterile. The foreign DNA sequence can also comprise at least one marker DNA that is under the control of, and is fused at its 5' end to, the second promoter and is fused at its 3' end to suitable transcription termination signals, including a polyadenylation signal. The marker DNA is preferably in the same genetic locus as the female-sterility DNA, whereby the second RNA, protein or polypeptide is produced in at least the specific tissue or specific cells of the female-sterile plant so that the plant can be easily distinguished and/or separated from other plants that do not contain the second RNA, protein or polypeptide in the specific tissue or specific cells. This guarantees, with a high degree of certainty, the joint segregation of both the female-sterility DNA and the marker DNA into offspring of the plant.

This invention relates to any plant, the nuclear genome of which can be transformed with a female-sterility DNA under the control of a first promoter that can direct expression of the female-sterility DNA selectively in the cells of the flowers, particularly a female organ thereof, whereby the plant can be both self-pollinated or cross-pollinated.

The cell of the plant (particularly a plant capable of being infected with Agrobacterium) is preferably transformed in accordance with this invention, using a vector that is a disarmed Ti-plasmid containing the foreign DNA sequence and carried by Agrobacterium. This transformation can be carried out using procedures described, for example, in European patent publications 0,116,718 and 0,270,822. Preferred Ti-plasmid vectors contain the foreign DNA sequence between the border sequences, or at least located to the left of the right border sequence, of the T-DNA of the Ti-plasmid. Of course, other types of vectors can be used to transform the plant cell, using procedures such as direct gene transfer (as described, for example, in European patent publication 0,223,247), pollen mediated transformation (as described, for example, in European patent publication 0,270,356, PCT publication WO85/01856, and European patent publication 0,275,069), in vitro protoplast transformation (as described, for example, in US patent 4,684,611), plant RNA virus-mediated transformation (as described, for example, in European patent publication 0,067,553 and US patent 4,407,956) and liposome-mediated transformation (as described, for example, in US patent 4,536,475).

Preferably, a nuclear female-sterile male-fertile plant of this invention is provided by transforming a plant cell with a disarmed Ti-plasmid vector containing the foreign DNA sequence with a female-sterility DNA under the control of a first promoter and preferably a marker DNA under the control of a second promoter. The marker DNA can be upstream or downstream of the female-sterility DNA in the Ti-plasmid vector, but preferably, the two are adjacent to one another and are located between the border sequences or at least located to the left of the right border sequence of the Ti-plasmid vector, so that they are properly transferred together into the nuclear genome of the plant cell. However, if desired, the cell can initially be transformed with a foreign DNA sequence containing a female-sterility DNA and a first promoter and can subsequently be transformed with a marker DNA and a second promoter, inserted into or near the genetic locus of the female-sterility DNA in the cell's nuclear genome, or this transformation can be carried out vice versa. Suitable vectors for this purpose are the same as those discussed above for transforming cells with the foreign DNA sequence. The preferred vector is a disarmed Ti-plasmid vector.

The selection of the female-sterility DNA of this invention is not critical. A suitable female-sterility DNA can be selected and isolated in a well-known manner, so that it encodes the first RNA, protein or polypeptide which significantly disturbs adversely the proper metabolism and/or functioning and/or development of any cell of a flower and/or seed and/or embryo in which the female-sterility DNA is expressed, preferably leading thereby to the death of such cell. Preferred examples of female-sterility DNAs encode: RNases such as RNase T1 (which degrades RNA molecules by hydrolyzing the bond after any guanine residue) and Barnase; DNases such as an endonuclease (e.g., EcoRI); or proteases such as a papain (e.g., papain zymogen and papain active protein). Other preferred examples of female-sterility DNAs encode: ribonucleases such as T₂ (Kawata et al (1988) Eur. J. Biochem 176, 683-697) or Rh (Horiuchi et al (1988) J. Biochem 103, 408-418); or glycoproteins such as are encoded by the S1, S2, S3, S6 and S7 alleles, particularly of Nicotiana alata (McClure et al (1989) Nature 342, 955-957).

Other examples of female-sterility DNAs encode enzymes which catalyze the synthesis of phytohormones, such as: isopentenyl transferase which is an enzyme that catalyzes the first step in cytokinin biosynthesis and is encoded by gene 4 of Agrobacterium T-DNA; or one or both of the enzymes involved in the synthesis of auxin and encoded by gene 1 and gene 2 of Agrobacterium T-DNA. Yet other examples of female-sterility DNAs encode: glucanases; lipases such as phospholipase A₂ (Verheij et al (1981) Rev. Biochem. Pharmacol. 91, 92-203); lipid peroxidases; or plant cell wall inhibitors. Still other examples of female-sterility DNAs encode proteins toxic to plants cells, such as a bacterial toxin (e.g., the A-fragment of diphtheria toxin or botulin).

Still another example of a female-sterility DNA is an antisense DNA: i) which encodes a strand of RNA complementary to a strand of RNA that is endogenous to, and naturally transcribed in, the cells of the flower, seed or embryo of the plant of this invention and ii) which is under the control of an endogenous promoter as described, for example, in European patent publication 0,223,399. Such an antisense DNA can be transcribed into an RNA sequence capable of binding to the coding and/or non-coding portion of an RNA, naturally produced in the cell of the flower, seed or embryo, so as to inhibit the translation of the naturally produced RNA. Examples of such an antisense DNA are the antisense DNAs of: the STMG-type genes, such as the STMG07 gene, the STMG08 gene, the STMG4B12 gene and the STMG3C9 gene of Example 2 herein; the KTI3 gene (Jofuku and Goldberg (1989) The Plant Cell 1, 1079-1093); a gene encoding a seed-specific storage protein, such as a 2S albumin (Krebbers et al (1988) Plant Physiol. 87, 859-866; Altenbach et al (1987) Plant Molecular Biol. 8, 239-250; (Scolfield and Crouch (1987) J. Biol. Chem. 262, 12202-12208); or a gene corresponding to cDNA clone pMON9608 (Gasser et al (1989) The Plant Cell 1, 15). Such antisense DNAs can be naturally expressed in flower, seed or embryo cells of the plant under the control of the endogenous promoter of the complementary endogenous DNA strand (or gene) of the plant, for example: in the style (with the antisense DNA of the STMG07, STMG08, STMG4B12 or STMG3C9 gene); in the embryo axis (with the antisense DNA of the KTI3 gene); in seeds (with the antisense DNA of a 2S albumin-encoding gene); and in ovule cells (with the antisense DNA of PMON9608).

A further example of a female-sterility DNA encodes a specific RNA enzyme (i.e., a so-called "ribozyme"), capable of highly specific cleavage against a given target sequence as described by Haseloff and Gerlach (1988 ) Nature 334, 585-591. Such a ribozyme is, for example, the ribozyme targeted against the RNA encoded by the STMG07 gene, the STMG08 gene, the STMG4B12 gene, the STMG3C9 gene, the KTI3 gene, a gene encoding a seed-specific storage protein such as a 2S albumin or the gene corresponding to cDNA clone pMON9608.

Still other examples of female-sterility DNAs encode products which can render the flowers, seeds and/or embryos susceptible to a specific disease, such as a fungus infection. Such a female-sterility DNA can be used in a plant, in which all other cells or tissues, in which the female-sterility DNA is not expressed, are resistant to the specific disease.

Yet another example of a female-sterility DNA comprises a combination of: 1) a first gene encoding a viral dependent RNA polymerase, such as TNV replicase (Meulewater et al (1990) Virology 177, 1-11), under the control of a first promoter of this invention; and 2) a negative strand (i.e., antisense DNA) of a second gene: i) which encodes a first protein or polypeptide of this invention that, when produced or overproduced in the plant cell of this invention, disturbs significantly cell metabolism, development and/or functioning, ii) which is fused at its 3′ end to a viral RNA replication recognition sequence or so-called "viral subgenomic promoter", such as the TNV subgenomic promoter (Lexis et al (1990) J. of Virology 64 (4), 1726-1733); and iii) which is fused at its 5′ end to, and under the control of, another suitable promoter, such as a first promoter of this invention, capable of directing expression of the negative strand in the plant cell of this invention. The viral subgenomic promoter sequence is specifically recognized by the viral dependent RNA polymerase encoded by the first gene. This recognition leads to the repeated replication of the negative strand of the second gene as a sense strand, which leads to the synthesis of the first protein or polypeptide. Both the first gene and the negative strand are provided in the nuclear genome of the plant cell of this invention. This can be achieved by one transformation event, by two consecutive transformation events, or by crossing a plant having the first gene inserted into its genome with a plant having the negative strand inserted into its genome.

By "foreign" with regard to the foreign DNA sequence of this invention is meant that the foreign DNA sequence contains a foreign female-sterility DNA and/or a foreign first promoter. By "foreign" with regard to a DNA, such as a female-sterility DNA and a first promoter, as well as a marker DNA, a second promoter and any other DNA in the foreign DNA sequence, is meant that such a DNA is not in the same genomic environment in a plant cell, transformed with such a DNA in accordance with this invention, as is such a DNA when it is naturally found in the cell of the plant, bacteria, animal, fungus, virus, or the like, from which such a DNA originates. This means, for example, that a foreign female-sterility DNA or marker DNA can be: 1) a nuclear DNA in a plant of origin; 2) endogenous to the transformed plant cell (i.e., from a plant of origin with the same genotype as the plant being transformed); and 3) within the same transcriptional unit as its own endogenous promoter and 3′ end transcription regulation signals (from the plant of origin) in the foreign DNA sequence of this invention in the transformed plant cell; but 4) inserted in a different place in the nuclear genome of the transformed plant cell than it was in the plant of origin so that it is not surrounded in the transformed plant cell by the genes which surrounded it naturally in the plant of origin. A foreign female-sterility or marker DNA can also, for example, be: 1) a nuclear DNA in a plant of origin; and 2) endogenous to the transformed plant cell; but 3) in the same transcriptional unit as a different (i.e., not its own) endogenous promoter and/or 3′ end transcription regulation signals in a foreign chimaeric DNA sequence of this invention in a transformed plant cell. A foreign female-sterility or marker DNA can also, for example, be: 1) a nuclear DNA in a plant of origin; and 2) endogenous to the transformed plant cell; but 3) in the same transcriptional unit as a heterologous promoter and/or 3′ end transcription regulation signals in a foreign chimaeric DNA sequence of this invention in a transformed plant cell. A foreign female-sterility or marker DNA can also, for example, be heterologous to the transformed plant cell and in the same transcriptional unit as an endogenous promoter and/or 3′ transcription regulation signals (e.g., from the nuclear genome of a plant with the same genotype as the plant being transformed) in a foreign chimaeric DNA sequence of this invention in a transformed plant cell. An example of a foreign female-sterility DNA could come from the nuclear genome of a plant with the same genotype as the plant being transformed and encode a catalytic enzyme, such as a protease or ribonuclease, that is endogenous to cells of the flowers, seeds and/or embryos of the plant being transformed, so that the enzyme is overproduced in transformed cells of the flowers, seeds and/or embryos in order to disturb significantly their metabolism, functioning and/or development. Preferably, the female-sterility DNA and the marker DNA are each heterologous to the plant cell being transformed.

By "heterologous" with regard to a DNA, such as a female-sterility DNA, a first promoter, a marker DNA, a second promoter and any other DNA in the foreign DNA sequence of this invention, is meant that such a DNA is not naturally found in the nuclear genome of cells of a plant with the same genotype as the plant being transformed. Examples of heterologous DNAs include chloroplast and mitochondrial DNAs obtained from a plant with the same genotype as the plant being transformed, but preferred examples are chloroplast, mitochondrial, and nuclear DNAs from plants having a different genotype than the plant being transformed, DNAs from animal and bacterial genomes, and chromosomal and plasmidial DNAs from fungal and viral genomes.

By "chimaeric" with regard to the foreign DNA sequence of this invention is meant that at least one of its female-sterility DNAs: 1) is not naturally found under the control of its first promoter for the one female-sterility DNA; and/or 2) is not naturally found in the same genetic locus as at least one of its marker DNAs. Examples of foreign chimaeric DNA sequences of this invention comprise: a female-sterility DNA of bacterial origin under the control of a first promoter of plant origin; and a female-sterility DNA of plant origin under the control of a first promoter of plant origin and in the same genetic locus as a marker DNA of bacterial origin.

By "flower" is meant to include the entire organ of a flower, as well as one or more of its individual parts such as its shoot axis, sepals, petals, male reproductive organs (or stamens) and female reproductive organs (or carpels), whose wholly or partly, retarded or arrested development in accordance with this invention prevents the development of viable seeds in the flower but not the development and propagation of its male gametes; by "female organ" is meant the entire organ of a flower that is involved in the production of female gametes and/or viable seeds and/or viable embryos, as well as one or more of its individual parts such as its ovule, ovary, style, stigma, corolla, disc, septum, calyx and placenta tissue. By "embryo" is meant to include the entire embryo of a plant, as well as one or more of its individual parts such as its embryo axis and embryo cotyledons.

So that the female-sterility DNA of this invention is expressed selectively in cells of the flowers, particularly one or more of their female organs, in cells of the seeds and/or in cells of the embryos of the plants of this invention, it is preferred that the first promoter, which controls the female-sterility DNA in the foreign DNA sequence, be a promoter capable of directing gene expression selectively in cells of the flowers, seeds and/or embryos of the plant. Such a flower-, seed- and/or embryo-specific promoter can be an endogenous promoter or an exogenous promoter and can be from the nuclear genome or from the mitochondrial or chloroplast genome of a plant cell. In any event, the first promoter is foreign to the nuclear genome of the plant cell, being transformed. Preferably, the first promoter causes the female-sterility DNA to be expressed only in cells of one or more specific tissues of the flowers, preferably one or more female organs thereof, or of the seeds or of the embryos, especially in style cells, ovary cells, septum cells, seedcoat cells, endosperm cells, embryo axis cells and/or embryo cotyledon cells.

The first promoter of this invention can be selected and isolated in a well known manner from a plant, to be rendered female-sterile, so that the first promoter directs expression of the female-sterility DNA selectively in cells of the flowers, seeds and/or embryos of the plant, so as to kill or disable the plant's flowers, seeds and/or embryos and render the plant incapable of producing fertile female gametes, viable seeds and/or viable embryos. The first promoter is preferably also selected and isolated so that it is effective to prevent expression of the female-sterility DNA in other parts of the plant that are not involved in the production of fertile female gametes, viable seeds and/or viable embryos, especially in male organs of the flowers, so that the plant remains male-fertile. For example, a suitable flower-specific (preferably female reproductive organ-specific), seed-specific or embryo-specific first promoter can be identified and isolated in a plant, to be made female-sterile, by:
1. searching for an mRNA which is only present in the plant during the development of its flowers, seeds or embryos, preferably its ovary, style, placenta, calyx, scutellum, septum, seedcoat, endosperm or embryo cotyledons;
2. isolating this flower-, seed- or embryo-specific mRNA;
3. preparing a cDNA from this specific mRNA;
4. using this cDNA as a probe to identify the regions in the plant genome which contain DNA coding for this specific mRNA; and then
5. identifying the portion of the plant genome that is upstream (i.e., 5′) from the DNA coding for this specific mRNA and that contains the promoter of this DNA.

Examples of a first promoter of this invention are the Nicotiana tabacum promoters of the STMG-type genes, described in Example 2, which are style and/or stigma specific promoters. Other style-stigma specific first promoters from other plant species can be isolated from their genomes, using the STMG-type genes as a probe as in step 4, above. Under hybridizing conditions, such a probe will hybridize to DNA coding for a style-stigma specific mRNA in a mixture of DNA sequences from the genome of the other plant species (Maniatis et al (1982) Molecular Cloning. A Laboratory Manual. Ed. Cold Spring Harbor Laboratory). Thereafter, as in step 5 above, another style-stigma specific first promoter can be identified. Other style-specific promotors can be isolated from self-incompatibility genes, such as an S-gene, for example as isolated from Nicotiana alata (McClure et al (1989) Nature 342, 955-957). Other female organ-specific promoters can be identified using other female organ-specific cDNAs, such as cDNA clone pMON9608 (Gasser et al (1989) The Plant Cell 1, 15) that hybridizes exclusively with a gene expressed only in the ovules of tomato plants.

Other examples of such a first promoter are: the promoter of the KTI3 gene (Jofuku and Goldberg (1989) The Plant Cell 1, 1079-1093) which is an embryo axis-specific promoter; and the seed-specific promoters derived from genes encoding seed-specific storage proteins, such as the PAT2S promoters, for example PAT2S1, PAT2S2, PAT2S3 and PAT2S4 which are promoters of the four 2S albumin genes ("AT2S genes") of Aribidopsis thaliana (Krebbers et al (1988) Plant Physiol. 87, 859-866).

If more than one female-sterility DNA is present in the foreign DNA sequence of this invention, all the female-sterility DNAs can be under the control of a single first promoter, but preferably, each female-sterility DNA is under the control of its own separate first promoter. Where a plurality of female-sterility DNAs are present in the foreign DNA sequence, each female-sterility DNA can encode the same or different first RNA, polypeptide or protein. For example, when the female-sterility DNA encodes an RNase such as RNase T1, it is preferred that at least 3, particularly 4 to 6, copies of the female-sterility DNA and its first promoter be provided in the foreign DNA sequence. In such a case it is also preferred that all the female-sterility DNAs and their first promoters be adjacent to one another in the foreign DNA sequence and in any vector used to transform plant cells with the foreign DNA sequence. If the plurality of female-sterility DNAs encode different products, such as gene 1 and gene 2 or such as TNV replicase and a RNase, DNase or protease, it may be preferred that the female-sterility DNAs not be adjacent to one another and perhaps not even be present in the same vector used to transform plant cells with the foreign DNA sequence or even not present in the same parent plant of the female-sterile plant of this invention.

The selection of the marker DNA of this invention also is not critical.

In this regard the marker DNA may be any DNA sequence encoding a second RNA, protein or polypeptide which confers on at least a specific plant tissue or specific plant cells, in which such DNA sequence is expressed, a distinctive trait compared to such a specific plant tissue or specific plant cells in which such DNA sequence is not expressed.

A suitable marker DNA can be selected and isolated in a well known manner, so that it encodes a second RNA, protein or polypeptide that allows plants or their tissue, seeds or even cells, expressing the marker DNA, to be easily distinguished and separated from plants or their tissue, seeds or even cells not expressing the second RNA, protein or polypeptide. Examples of marker DNAs encode proteins that can provide a distinguishable color to plant cells, such as the A1 gene encoding dihydroquercetin-4-reductase (Meyer et al (1987) Nature 330, 677-678) and the glucuronidase gene (Jefferson et al (1988) Proc. Natl. Acad. Sci. USA ("PNAS") 83, 8447), or that provide a specific morphological characteristic to the plant such as dwarf growth or a different shape of the leaves. Other examples of marker DNAs confer on plants: stress tolerance, such as is provided by the gene encoding superoxide dismutase as described in European patent application 88/402222.9; disease or pest resistance such as is provided by a gene encoding a Bacillus thuringiensis endotoxin conferring insect resistance as described in European patent application 86/300291.1 or a gene encoding a bacterial peptide that confers a bacterial resistance as described in European patent application 88/401673.4.

Preferred marker DNAs encode second proteins or polypeptides inhibiting or neutralizing the action of herbicides such as: the sfr gene and the sfrv gene encoding enzymes conferring resistance to glutamine synthetase inhibitors such as bialaphos and phosphinotricine as described in European patent application 87/400,544.0; and genes encoding modified target enzymes for certain herbicides that have a lower affinity for the herbicides than naturally produced endogenous enzymes, such as a modified glutamine synthetase as a target for phosphinotricine as described in European patent publication 0,240,792 and a modified 5-enolpyruvylshikimate-3 phosphate synthase as a target for glyphosate as described in European patent publication 0,218,571. Other examples are: marker DNAs encoding proteins which neutralize the action of the herbicide bromoxynil (Stalker et al (1988) in: Genetic Improvements of Agriculturally Important Crops, Ed: R.T. Fraley, N.M. Frey and J. Schell, Cold Spring Harbor Laboratories); the herbicide sulfonylurea (Lee et al (1988) EMBO J. 7, 1241-1248); and the herbicide 2,4 D (presented at the 2nd International Symposium of Plant Molecular Biology, Jerusalem, 13-18 November 1988).

The second promoter of this invention, which controls the marker DNA, can also be selected and isolated in a well known manner so that the marker DNA is expressed either selectively in one or more specific tissues or specific cells or constitutively in the entire plant, as desired depending on the nature of the second RNA, protein or polypeptide encoded by the marker DNA. For example, if the marker DNA encodes an herbicide resistance, it may be useful to have the marker DNA expressed in all cells of the plant, using a strong constitutive second promoter such as a 35S promoter (Odell et al (1985) Nature 313, 810-812), a 35S′3 promoter (Hull and Howell (1987) Virology 86, 482-493), the promoter of the nopaline synthetase gene ("PNOS") of the Ti-plasmid (Herrera-Estrella (1983) Nature 303, 209-213) or the promoter of the octopine synthase gene ("POCS" [De Greve et al (1982) J. Mol. Appl. Genet. 1 (6), 499-511]). If the marker DNA encodes a protein conferring disease resistance, it may be useful to have the marker DNA selectively expressed in wound tissue by using, for example, a TR promoter such as the TR1′ or TR2′ promoter of the Ti-plasmid (Velten et al (1984) EMBO J. 3, 2723-2730). If the marker DNA encodes a herbicide resistance, it also may be useful to have the marker DNA selectively expressed in green tissue by using, for example, the promoter of the gene encoding the small subunit of Rubisco (European patent application 87/400,544.0). If the marker DNA encodes a pigment, it also may be useful to have the marker DNA expressed in specific cells such as petal cells, leaf cells or seed cells, preferably in the outside layer of the seed coat.

One can identify and isolate in a well known manner a tissue-specific second promoter for a plant to be rendered female-sterile and easily distinguishable from non-transformed plants by:
1. searching for an mRNA which is only present in the plant during the development of a certain tissue, such as its petals, leaves or seeds;
2. isolating this tissue-specific mRNA;
3. preparing a cDNA from this tissue-specific mRNA;
4. using this cDNA as a probe to identify the regions in the plant genome which contain DNA coding for the tissue-specific mRNA; and then
5. identifying the portion of the plant genome that is upstream from the DNA coding for the tissue-specific mRNA and that contains the promoter for said DNA.

If more than one marker DNA is present in the foreign DNA sequence of this invention, all the marker DNAs can be under the control of a single second promoter, but preferably, each marker DNA is under the control of its own separate second promoter. More preferably, each marker DNA is under the control of its own second promoter and encodes a different second RNA, protein or polypeptide, providing different distinguishable characteristics to a transformed plant. In some cases, it may be preferred that the marker DNA(s) and second promoter(s) are adjacent to each other and to the one or more female-sterility DNAs contained in the foreign DNA sequence of this invention and in any vector used to transform plant cells with the foreign DNA sequence. In other cases, it may be preferred that the marker DNAs are not adjacent to each other and/or to the female-sterility DNAs.

It is generally preferred that the first RNA, protein or polypeptide, encoded by the female-sterility DNA, interferes significantly with the metabolism, functioning and/or development of the cells of the flowers and/or seeds and/or embryos by acting in the cytoplasm or the nucleus of these cells. However, when it is desired to have the first protein or polypeptide and/or the second protein or polypeptide transported from the cytoplasm into chloroplasts or mitochondria of the cells of transformed plants, the foreign DNA sequence can further include a first additional foreign DNA encoding a transit peptide. The first additional DNA is located between the female-sterility DNA and the first promoter if the first protein or polypeptide is to be so-transported and is between the marker DNA and the second promoter if the second protein or polypeptide is to be so-transported. By "transit peptide" is meant a polypeptide fragment which is normally associated with a chloroplast or mitochondrial protein or subunit of the protein that is produced in a cell as a precursor protein encoded by the nuclear DNA of the cell. The transit peptide is responsible for the translocation process of the nuclear-encoded chloroplast or mitochondrial protein or subunit into the chloroplast or the mitochondria, and during such a process, the transit peptide is separated or proteolytically removed from the chloroplast or mitochondrial protein or subunit. One or more of such first additional DNAs can be provided in the foreign DNA sequence of this invention for transporting one or more first or second proteins or polypeptides as generally described in European patent applications 85/402,596.2 and 88/402,222.9 and in: Van den Broeck et al (1985) Nature 313, 358-363; Schatz (1987) Eur. J. of Bioch. 165, 1-6; and Boutry et al (1987) Nature 328, 340-342. An example of a suitable transit peptide for transport into chloroplasts is the transit peptide of the small subunit of the enzyme RUBP carboxylase (European patent application 85/402,596.2), and an example of a transit peptide for transport into mitochondria is the transit peptide of the enzyme Mn-superoxide dismutase (see example 10 herein and European patent application 89/401,194.9).

It is also generally preferred that the first RNA, protein or polypeptide, encoded by the female-sterility DNA, act intracellularly so as to interfere with cell metabolism, functioning and/or development in the plant. However when it is desired to have the first protein or polypeptide and/or the second protein or polypeptide secreted out of the intercellular areas of the plant cells, in which they are expressed, or out of the tissue, in which they are expressed, the foreign DNA sequence can further include a second additional foreign DNA encoding a secretory signal peptide. The second additional foreign DNA is located between the female-sterility DNA and the first promoter if the first protein or polypeptide is to be secreted and between the marker DNA and the second promoter if the second protein or polypeptide is to be secreted. By "secretory signal peptide" is meant a natural polypeptide fragment which is, particularly in eukaryotic cells, associated during translocation with proteins that are normally secreted from cells or an artificial polypeptide fragment which, when associated during translocation with a protein or polypeptide, provokes its secretion from cells. Examples of suitable secretory signal peptides are set forth in: Von Heijne (1986), NAR 14 (11), 4683-4690; Denecke et al (1990), The Plant Cell 2, 51-59.

In the foreign DNA sequence of this invention, 3' transcription termination and polyadenylation signals can be selected in a conventional manner from among those which are capable of providing correct transcription termination and polyadenylation of mRNA in plant cells. The transcription termination and polyadenylation signals can be the natural ones of the gene to be transcribed but can also be foreign or heterologous. Examples of heterologous transcription termination and polyadenylation signals are those of the octopine synthase gene (Gielen et al (1984) EMBO J. 3, 835-845) and the T-DNA gene 7 (Velten and Schell (1985) Nucleic Acids Research ("NAR") 13, 6981-6998).

Also in accordance with this invention, plant cell cultures, containing the foreign DNA sequence of this invention, can be used to regenerate homozygous dominant female-sterile male-fertile plants by performing the necessary transformations on diploid (Chuong and Beversdorf (1985) Plant Sci. 39, 219-226) or on haploid cell cultures and then (for haploid cell cultures) doubling the number of chromosomes by well known techniques (e.g., with colchicine). See: Plant Tissue and Cell Culture, Plant Biology 3, A.R. Liss, Inc. N.Y. (1987). Thereby, the foreign DNA sequence will be in homozygous form in the nuclear genome of each of the so-transformed plant cells. This is preferred for plant cell cultures containing a female-sterility DNA under the control of a first promoter which directs gene expression at a given stage of development of the female gametes, such as ovums, especially after meiosis, or in cells derived from the female gametes, such as seed or embryo cells, so that the female-sterility DNA is present and can be expressed in all female gametes or plant cells derived therefrom.

Also in accordance with this invention, processes are provided for producing hybrid seeds which can be grown into hybrid plants. These processes involve crossing in an otherwise conventional manner: a) a nuclear female-sterile male-fertile plant of this invention, in which the first RNA, protein or polypeptide is expressed selectively in flowers, preferably in at least one female organ thereof, or in embryos; with b) a male-sterile female-fertile plant. Suitable male-sterile female-fertile plants are described in European Patent Application 89/401194.9 as having a nuclear genome, in which are stably integrated:
(a) a male-sterility DNA encoding a RNA, protein or polypeptide which, when produced or overproduced in a stamen cell of a plant, significantly disturbs adversely the metabolism, functioning and/or development of the stamen cell; and
(b) a promoter capable of directing expression of the male-sterility DNA selectively in stamen cells of the plant, preferably in anther, pollen and/or filament cells, particularly in tapetum and/or anther epidermal cells; the male-sterility DNA being in the same transcriptional unit as, and under the control of, this promoter;
and which optionally has in the same genetic locus:
(c) another marker DNA encoding a RNA, protein or polypeptide which, when present at least in a specific tissue or in at least specific cells of the plant, renders the plant easily separable from other plants which do not contain this RNA, protein or polypeptide at least in the specific tissue or specific cells; and
(d) another promoter capable of directing expression of the other marker DNA at least in the specific tissue or specific cells and being in the same transcriptional unit as, and controlling, the other marker DNA.
The female-sterile plants and male-sterile plants are planted at random, near to each other to increase the chances of cross-pollination, without the need for precise planting patterns. The harvested seed, which is capable of germinating, will be the result of the fertilization of the male-sterile plants by the female-sterile plants and will be 100% hybrid. When the foreign DNA sequences responsible for the female-sterility and male-sterility characteristics are present in heterozygous form in the nuclear genomes of the respective parent plants, plants grown from such hybrid seed will be: 25 % fertile, 25 % female-sterile, 25 % male-sterile and 25 % sterile. When the foreign DNA sequence encoding female-sterility is present in the nuclear genome of the male-fertile parent plant in homozygous form--which is preferred when the first promoter is an ovum-, seed- or embryo-specific promoter--all the plants grown from such hybrid seed will be female-sterile.

Further in accordance with this invention, processes are provided for producing fruit without seeds by crossing in an otherwise conventional manner: a) a nuclear female-sterile male-fertile plant of this invention, in which the first RNA, protein or polypeptide is expressed selectively in seeds and in which the foreign DNA sequence, encoding the first RNA, protein or polypeptide, is preferably in homozygous form in the nuclear genome of the plant; with b) a male-sterile female-fertile plant.

Plants, transformed with the female-sterility DNA and in some cases preferably also with the marker DNA encoding an herbicide resistance, stably integrated in the plants' nuclear genomes and transmissible throughout generations as dominant alleles in accordance with this invention, are alternatives to, and can provide advantages over, presently used cytoplasmic and nuclear male-sterility systems for breeding and producing hybrid crops. In this regard, female-sterile male-fertile plants can provide: 1) inhibited seed formation in crops, 2) hybrid seeds for crops which do not easily cross-pollinate, and 3) easier breeding of plant lines as discussed below.

### 1. Inhibition of seed formation

There exist a wide variety of crops cultivated by man in which the seed is an undesirable by-product.
a) When the economic product of a plant consists of its vegetative part. By inhibiting seed production, the plant's energy can be focused on vegetative biomass production. Examples are perennial plants (e.g. forage grasses, forage legumes and rubber trees), some annual plants (e.g., sugar cane and potato), and especially all crops that would normally flower and set seed before the economic product is harvested. Other examples are plants, obtainable through genetic engineering, which produce, within their vegetative tissues, proteins, polypeptides or other metabolites for pharmaceutical or industrial purposes.
b) When the economic product of a plant is its fruit and it is desirable that the fruit be seedless, either because of consumer preferences (e.g., in tomato, melon and citrus fruit) or because seed formation uses up biomass that could otherwise be stored in the fruit (e.g., for providing high solids in tomatoes to be processed). Since such crops require fruit formation, the seedless condition, which usually induces fruit abortion, has to be compensated for by the possibility of obtaining parthenocarpic fruit set. Natural parthenocarpic fruit inducing genes exist in some crops, such as tomato and melon.
c) When the plant is not grown for its seeds, but remaining propagules after harvesting may give rise to seed formation. The regrowth from these seeds can cause a considerable weed problem in the next culture. This "weed" problem is particularly well known with sugar beet.
d) When the plant is grown for its flowers (e.g., cut flowers, pot plants or garden ornamentals). For these species, it is often desirable to avoid seed set. In the case of cut flowers or pot plants, fertilization of the flowers often induces an accelerated senescence of the petals. In case of garden ornamentals, the formation of fruits and seeds reduces the time span and the intensity of flowering.

### 2. Hybrid seed production

Engineered female-sterility is useful as a seed production tool in combination with natural cytoplasmic or nuclear male-sterility systems or engineered nuclear male-sterility systems for the production of commercial hybrid seeds in crops where the seed is not the economic harvest and which do not easily cross-pollinate (e.g., for forage grasses, forage legumes, sugar beet, and many vegetables). The breeding of nuclear female-sterile plants with male-sterile plants provides a better control of hybrid seed quality (e.g., no mistakenly harvested male rows) and a higher seed set by favoring cross-pollination through at random interplanting of male-sterile and female-sterile parent plants and does not need the use of a restorer of fertility. A strategy for such production of hybrid seeds (e.g., for sugar-beet) may include the following steps ("MS" stands for male-sterility, "FS" stands for female-sterility and "H" stands for herbicide resistance):

### A. Development of the female parent line A

Aa) Transform line A with a foreign DNA including a male-sterility DNA under the control of a stamen specific promoter and adjacent thereto a marker DNA encoding herbicide resistance, according to European patent application 89/401,194.9, giving A^{MSH/msh}
Ab) Maintain line A^{MSH/msh} through crossing with line A^{msh/msh}. This gives:
   50% A^{MSH/msh} (male-sterile, herbicide resistant) and
   50% A^{msh/msh} (fertile, herbicide sensitive).

### B. Development of the male parent line B

Ba) Transform line B with the chimaeric DNA sequence of this invention including a female-sterility DNA under the control of a first promoter which directs gene expression selectively in cells of a female organ of the plant and adjacent thereto a marker DNA encoding herbicide resistance, giving B^{FSH/fsh}.
Bb) Maintain line B^{FSH/fsh} through crossing with B^{fsh/fsh}, yielding:
   50% B^{FSH/fsh} (female-sterile,herbicide resist.) and
   50% B^{fsf/fsh} (fertile, herbicide sensitive).

### C. Producing the hybrid seed crop

Ca) Planting seeds obtained in Ab) and Bb) at random.
Cb) Eliminating through spraying with the herbicide the undesirable genotype before cross- and self-pollination could occur.
Cc) Cross-pollination occurring:
   A^{MSH/msh} x B^{FSH/fsh}
   giving 100% hybrid seeds with the following genotype:
   25% AB^{MSH/msh; FSH/fsh}
   25% AB^{MSH/msh; fsh/fsh}
   25% AB^{msh/msh; FSH/fsh}
   25% AB^{msh/msh; fsh/fsh}

This represents the commercially sold seed.

### 3. Easier Breeding

### a) without a marker DNA

The ability to obtain microspore-derived double haploids of most major crops allows the production of homozygous nuclear female-sterile lines in a more or less straightforward way (Chuong and Beversdorf (1985) Plant Sci. 39, 219-226). This makes it unnecessary in many cases to have a marker DNA within the same genetic locus of the nuclear genome of the cells of the plant as the female-sterility DNA. This is especially so if the homozygous female-sterile plant can be vegetatively multiplied (e.g., many vegetables).

### b) with a marker DNA

In case the female-sterility DNA is in the same genetic locus of the nuclear genome of the transformed plant as a marker DNA (e.g., encoding herbicide resistance), homozygous female-sterile plants are technically superior to many other lines as tester parents in line evaluation programs. This is especially the case for crops where the seed is not the economic harvest and which can easily cross-pollinate. Indeed, these female-sterile plants allow the testing of many female- and male-fertile lines in close proximity to one another while making it easy to eliminate any self-pollinated seed from the different plant lines, being tested, from seeds resulting from crosses between the different lines.

The following Examples illustrate the invention. The figures referred to in the Examples are as follows:
Fig. 1A shows the cDNA sequence of the STMG07 gene of Example 1.
Fig. 1B shows the cDNA sequence of the STMG08 gene of Example 1.
Fig. 2A shows the cDNA sequence of the STMG4B12 gene of Example 1.
Fig. 2B shows the cDNA sequence of the STMG3C9 gene of Example 1.
Fig. 3 shows a map of the vector pMG100 of Example 4.
Fig. 4 shows a map of the vector pMG101 of Example 6.
Fig. 5 shows a map of the vector pMG102 of Example 8.
Fig. 6 shows a map of the vector pMG103 of Example 8.
Fig. 7 shows a map of the vector pMG104 of Example 10.

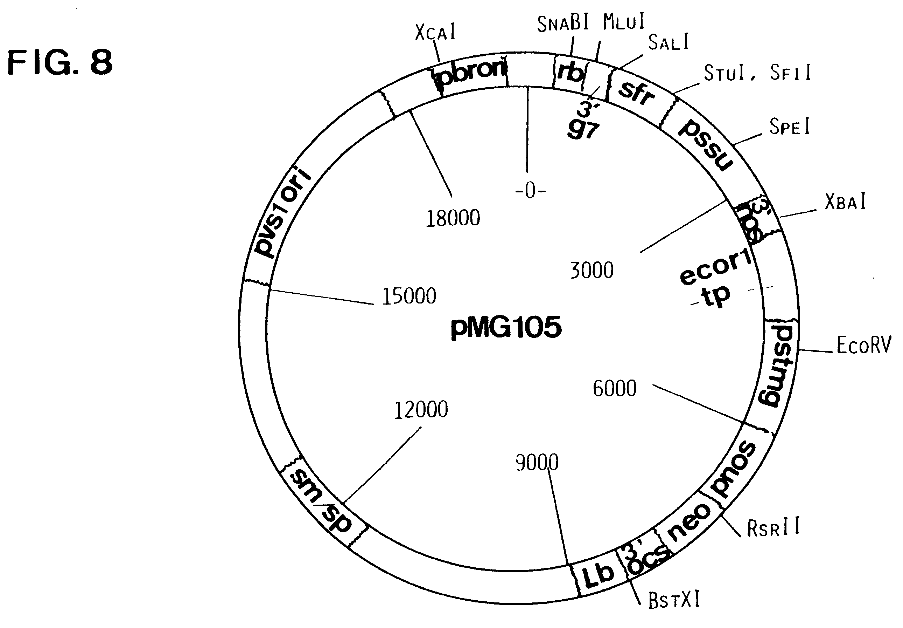

Fig. 8 shows a map of the vector pMG105 of Example 10.

Unless otherwise stated in the Examples, all procedures for making and manipulating recombinant DNA were carried out by the standardized procedures described in Maniatis et al, Molecular Cloning - A Laboratory Manual, Cold Spring Harbor Laboratory (1982). The following vector, used in the Examples, has been deposited in the Deutsche Sammlung Für Mikroorganismen und Zellculturen ("DSM"), Mascheroder Weg 1B, D-3300 Braunschweig, Federal Republic of Germany under the provisions of the Budapest Treaty:

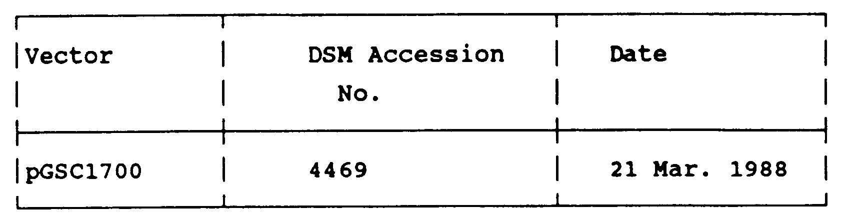

### Example 1 - Isolation of style-stigma specific cDNAs from Nicotiana tabacum "Petit Havana" SR1.

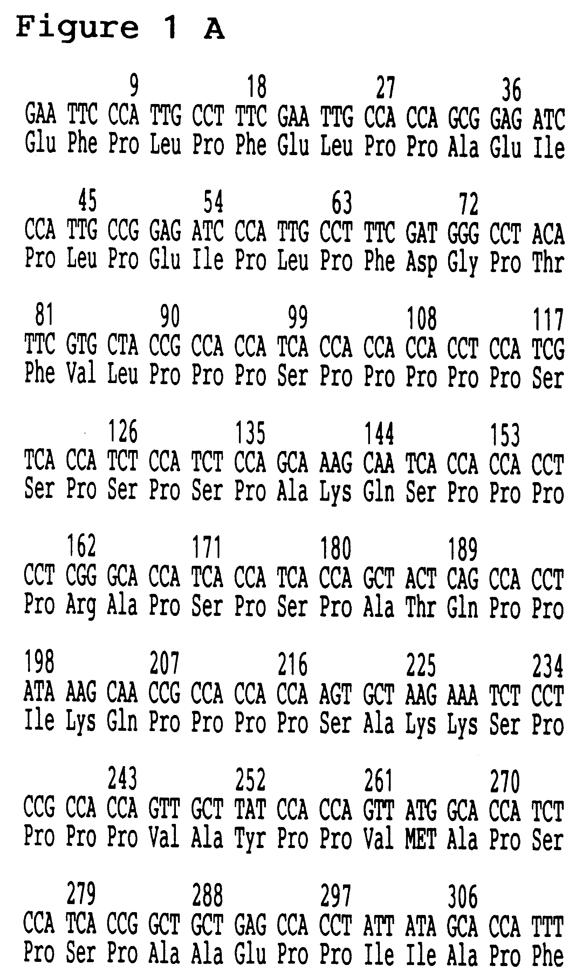

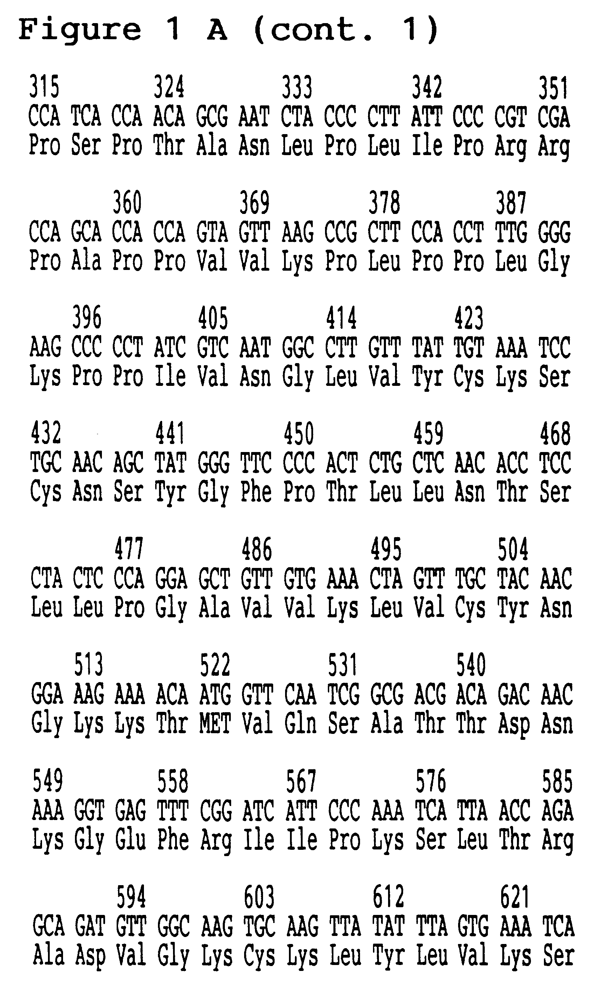

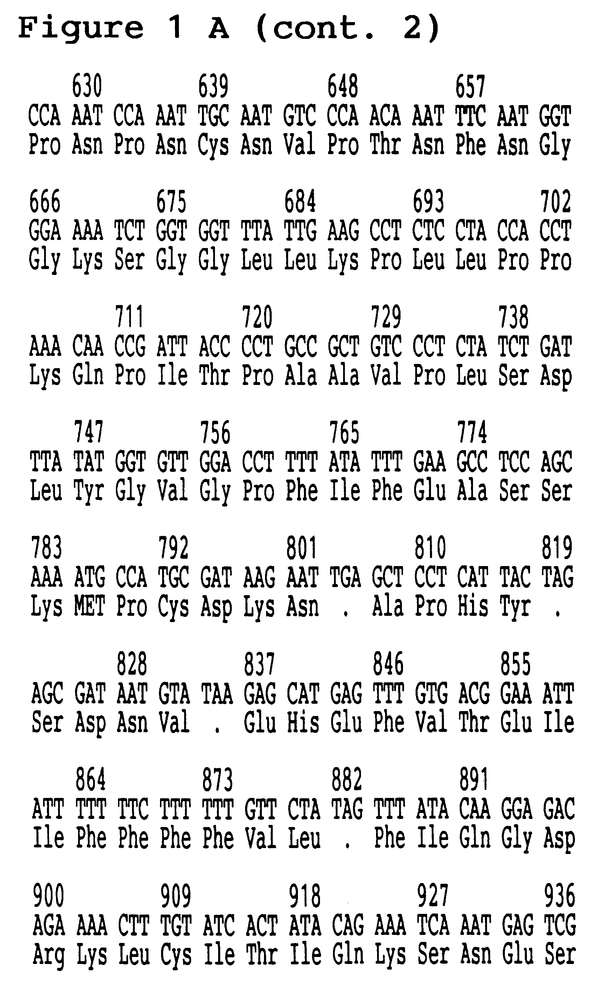

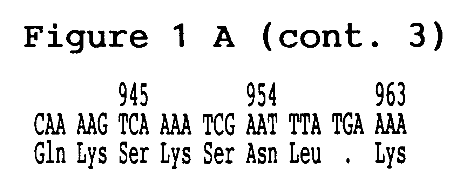

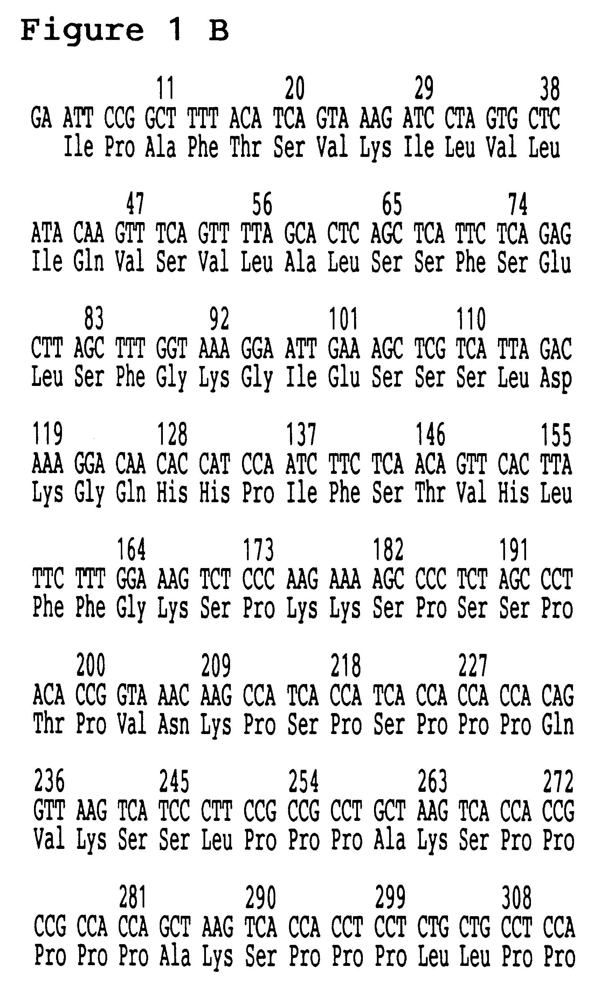

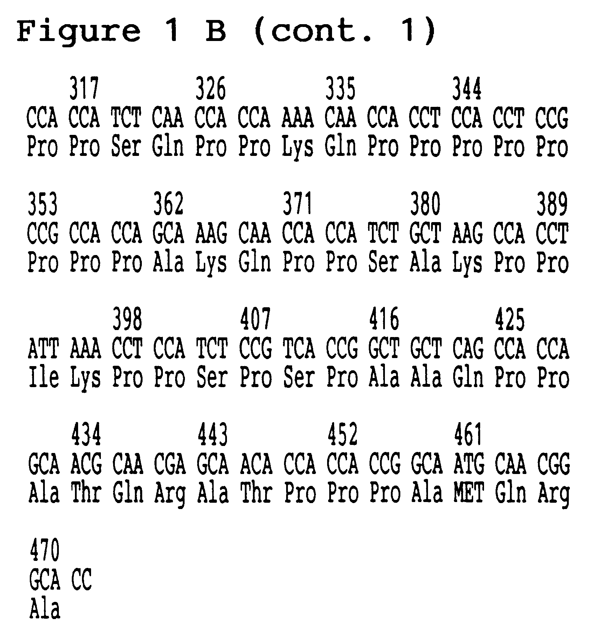

Using well known procedures (Maniatis et al, 1982), total mRNA was isolated from the following different tobacco tissues: style-stigma tissues from flowers in stage 3 to 7 (according to Goldberg (1988) Science 240, 1460-1467); so-called "young stage" style-stigma tissues from a flower which did not develop pollen grains in stage 8 to 11 (according to Goldberg, 1988); so-called "old stage" ovary tissue from flowers in young stage; ovary tissue from flowers in old stage; and stem, root and leaf tissue from in vitro cultivated seedlings. cDNAs were synthesized from young and old style-stigma tissues using the Amersham (Amersham International PLC, Buckinghamshire, England) kit, cDNA Synthesis System Plus-RPN 1256 Y/Z, according to the directions set forth in the kit for its use. The cDNAs were cloned in Lambda gt 10 vector using the Amersham kit, cDNA Cloning System-lambda gt 10 - RPN 1257, according to the directions set forth in the kit. From the cDNA library thus obtained, differential screening was performed with a cDNA probe from seedlings on the one hand and a cDNA probe from style-stigma tissues on the other hand. The selected clones were subcloned in pGEM1 (Promega, Madison, Wisconsin, USA). Probes of each of these subclones were prepared and first checked for their specificity in Northern blots with 10 ug of total mRNA from different tobacco tissues (roots, stems, leaves, sepals, petals, anthers, young stage style-stigma, old stage style-stigma, and old stage ovaries). The subclones, that specifically hybridized in these Northern blots with style-stigma mRNA, were again hybridized in Northern blots with 2 ug poly A⁺ mRNA isolated from the above-mentioned tissues, including young ovaries, seeds and virus-infected leaves. The clones, called "pMG07" and "pMG08" and containing an insert of 0.963 kb and 0.472 kb, respectively, proved to be style-stigma specific cDNA sequences. These clones were sequenced, and their cDNA sequences are shown in Fig. 1A and Fig. 1B, respectively. The DNA sequence of pMG07 reveals the presence of one open reading frame ("ORF") over a sequence of 800 nucleotides. The sequence of pMG08 reveals an ORF over the total sequence.

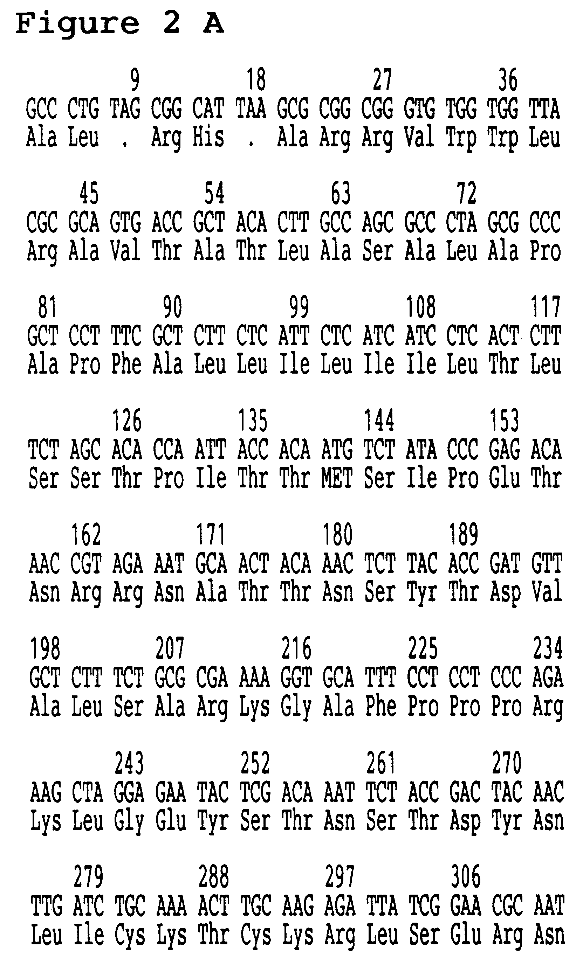

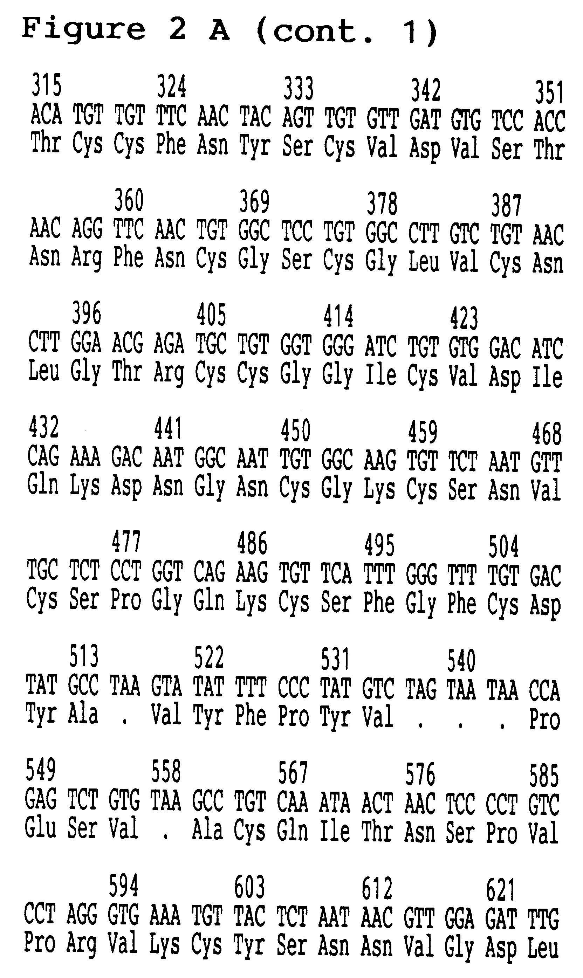

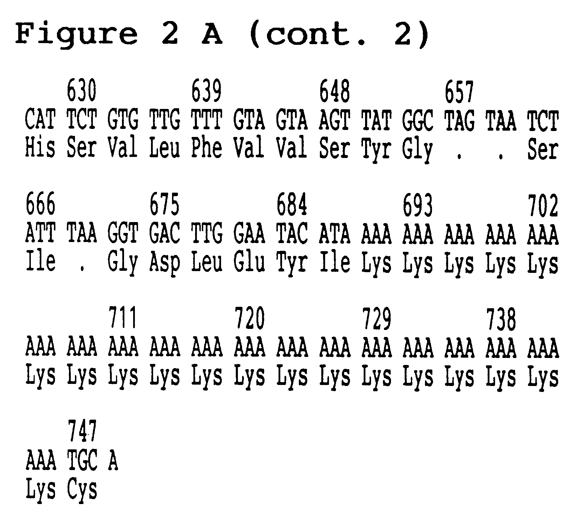

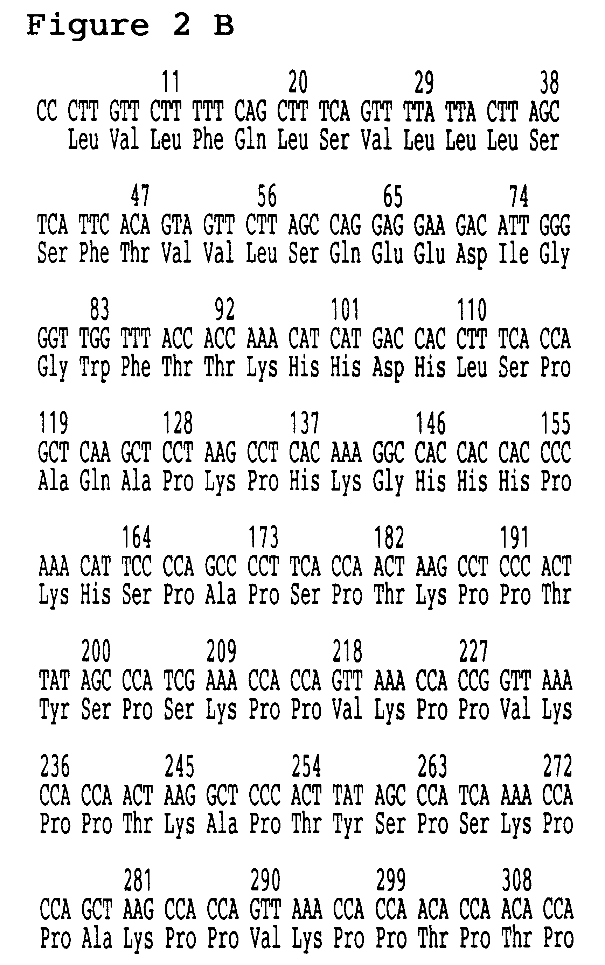

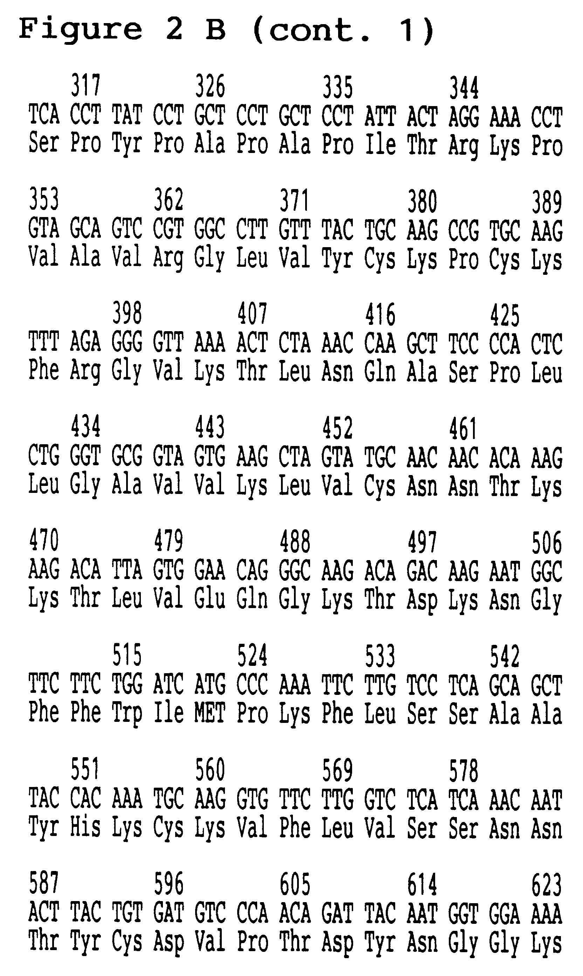

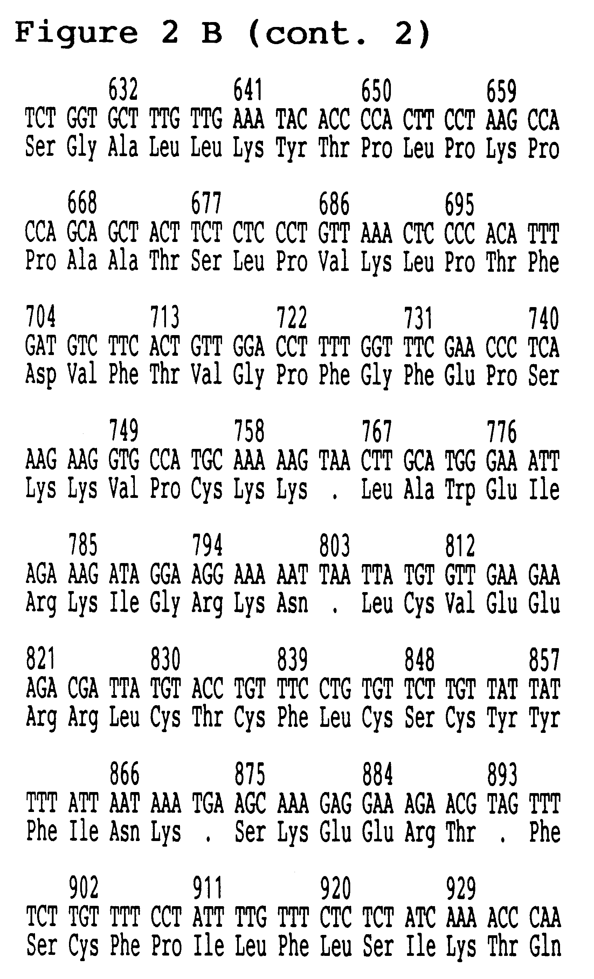

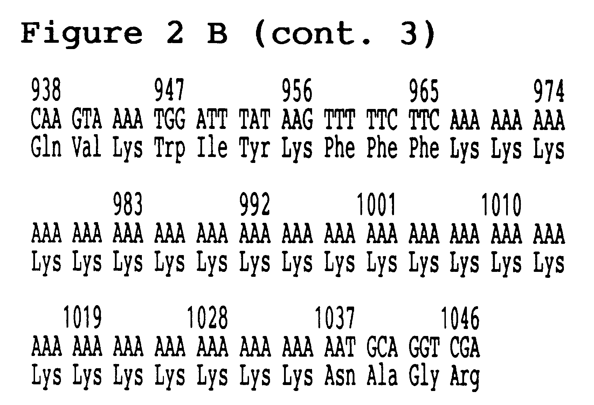

From Prof. Goldberg of the University of California, Los Angeles (UCLA) were obtained: two Nicotiana tabacum style-stigma specific cDNAs (4B12 and 3C9) cloned as a PstI - SmaI fragment in pGEM 3zf (-) (Promega, Madison, Wisconsin, USA). These clones contained inserts of 0.748 kb and 1.046 kb, respectively. Probes of these two clones were hybridized in Northern blots with 10 ug of total mRNA from different tobacco tissues (roots, stems, leaves, sepals, petals, anthers, young stage style-stigma, old stage style-stigma, and old stage ovaries) in order to check their specificity. These Northern blots confirmed the specificity of the clones and revealed that the transcript of 4B12 is 0.8 kb and that of 3C9 is 1.2 kb. The two clones were subcloned in pGEM1 (Promega), which subclones were called "pMG4B12" for the 4B12 clone and "pMG3C9" for the 3C9 clone. The subclones were again checked for their specificity in Northern blots with 2 ug poly A⁺ mRNA isolated from the above-mentioned tissues, including young ovaries, seeds and virus-infected leaves. The clones pMG4B12 and pMG3C9, containing inserts of 0.748 kb and 1.046 kb, respectively, proved to be style-stigma specific sequences. These clones were sequenced and their cDNA sequences are shown in Fig. 2A and Fig. 2B, respectively.

### Example 2 - Isolation of the style-stigma specific genes ("STMG-type genes") corresponding to the style-stigma cDNA clones

Using known procedures (Maniatis et al, 1982), a probe from each of the cDNA clones of Example 1 of style-stigma specific sequences was used to isolate the corresponding genomic gene sequence which is specifically expressed in style-stigma tissues of the female organ of tobacco. According to protocols provided by Promega, tobacco genomic DNA was partially digested with Sau3A, and the restriction fragments were cloned into the lambda phage vector GEM.12 (Promega), digested with Xho I to produce genomic clones called "lambda STG07", "lambda STG08", "lambda STG4B12" and "lambda STG3C9". Subsequently, these genomic clones were subcloned in pGEM1 (Promega) according to the procedure of Promega. The subclones were again analyzed by Southern blot, using the respective cDNA clones as probes in order to identify the clones which contained the style-stigma specific DNA sequences. These subclones, called respectively "pSTG07", "pSTG08", "pSTG4B12" and "pSTG3C9" were sequenced (Maxam and Gilbert (1977) PNAS 74, 560). The orientation of these clones was determined by Northern blot analysis with riboprobes of both senses. Comparison of each cDNA sequence with its respective genomic clone sequence led to the identification of the region of homology. At the 5′ end of each region, the ATG codon and the consensus sequence TATA were determined. That the "TATA" box is part of the promoter of the gene is confirmed by primer extention (Mcknight et al (1987) Cell 25, 385). The style-stigma specific genes, isolated using the style-stigma cDNA as probe, are called in general "STMG-type" genes. The style-stigma specific gene of pSTG07 is called "STMG07", that of pSTG08 is called "STMG08", that of pSTG4B12 is called "STMG4B12" and that of pSTG3C9 is called "STMG3C9".

### Example 3 - Construction of promoter cassettes ("PSTMG") derived from the respective STMG-type genes

To construct chimaeric DNA sequences containing 5' regulator sequences, including the promoter of an STMG-type gene, in the same transcriptional unit as, and controlling, a first heterologous female-sterility DNA, cassettes are constructed by subcloning a DNA fragment including a promoter into the polylinker of the pMAC5-8 vector system (European patent application 87/402348.4). This produces respective vectors which can be used to isolate single strand DNA for use in site directed mutagenesis.

Using site directed mutagenesis (European patent application 87/402348.4), the sequence surrounding the ATG initiation codon of each of the genes is modified in such a way that the mutation creates a given sequence which is a unique recognition site for a given restriction enzyme. The resulting plasmids each contain the newly created restriction site. The precise nucleotide sequence spanning the newly created restriction site is determined in order to confirm that it only differs from the 5' sequence of the corresponding STMG-type gene by the substitution, creating the new restriction site. The newly created promoter cassettes, each comprising a promoter, a 5' untranslated end of an STMG-type gene to its ATG initiation codon, and a new restriction site, are generally called "PSTMGs". The PSTMG containing the promoter and 5′ end of STMG07 is called "PSTMG07", that of STMG08 is called "PSTMG08", that of STMG4B12 is called "PSTMG4B12" and that of STMG3C9 is called "PSTMG3C9".

### Example 4 - Construction of chimaeric DNA sequences of a PSTMG and a RNAse T1 gene

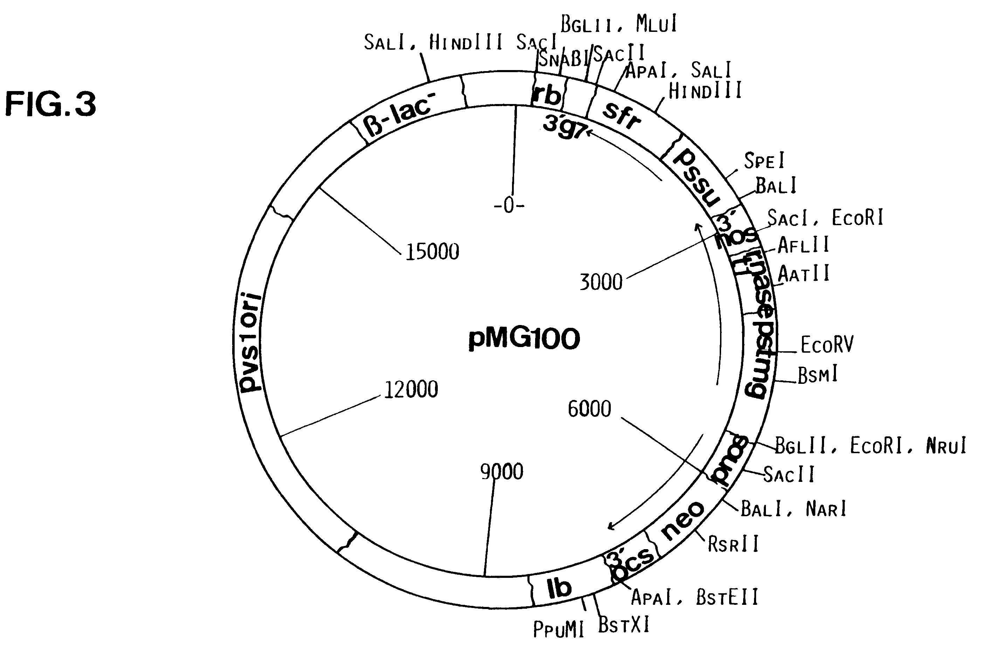

Plasmids named "pMG100", shown in Fig. 3, are constructed, each by assembling the following well known DNA fragments with a different one of the PSTMGs of Example 3:
1. a vector fragment, including T-DNA border sequences, from pGSC1700 in which the β-lactamase gene encoding ampicillin has been inactivated by insertion into the SacI site; located between the border sequences are the following DNA fragments 2-4;
2. a chimaeric sequence containing an Arabidopsis Rubisco SSU promoter ("PSSU" or "PSSUARA"), a herbicide resistance gene sfr (European patent application 87/400,544.0) and the 3′ end (i.e., transcription termination) signals of a T-DNA gene 7 (Velten and Schell (1985) NAR 13, 6981);
3. a chimaeric sequence containing the EcoRI/SacI fragment from pGSFR401 which contains a nopaline-synthase promoter ("PNOS"), a neo gene encoding kanamycin resistance and the 3′ end signals of an octopine synthase ("OCS") gene (European patent application 87/400,544.0, wherein pGSFR401 is called "pGSR4"); and
4. a chimaeric sequence, containing one of the PSTMG promoter cassettes from Example 3, fused in frame with a synthetic gene encoding RNase T1 from A. orhyzae, (Quaas et al, "Biophosphates and their Analogues-Synthese, Structure, Metabolism and Activity" (1987) Elsevier Science Publisher B.V., Amsterdam; Quaas et al (1988) Eur. J. Biochem. 173, 617-622) and the 3' end signals of a nopaline synthase ("NOS") gene (An et al (1985) EMBO J. 4 (2), 277).

Each pMG100 is a binary type T-DNA vector containing, within the T-DNA border sequences, three chimaeric sequences: PSSU-sfr and PNOS-neo which are marker DNAs with respectively PSSU and PNOS as second promoters; and PSTMG-RNase T1 gene which is a female-sterility DNA under the control of a PSTMG as a first promoter. Expression of the female-sterility DNA under the control of the PSTMG promoter will produce RNase T1 selectively in style and/or stigma cells. This will be lethal for the style and/or stigma cells since the RNase T1 will degrade the RNA molecules which are indispensable for these cells' metabolism.

### Example 5 - Introduction of each chimaeric DNA sequence of Example 4 into tobacco and alfalfa

A recombinant Agrobacterium strain is constructed by mobilization of each pMG100 (from Example 4) from E. coli into Agrobacterium tumefaciens C58C1 Rif^{R} containing pMP90 (Koncz and Schell (1986) Mol. Gen. Genetics 204, 382-396).

The resulting Agrobacterium strain, harboring pMP90 and pMG100, is used for the transformation of tobacco leaf discs (N. tabacum Petite Havane SR1), using standard procedures as described, for example, in European patent application 87/400,544.0, and of alfalfa according to the procedure described in D'Halluin et al (1990) Crop Science 30:866-871. Carbenicillin is used to kill the Agrobacterium strains after co-cultivation. Transformed calli are selected on a substrate containing 5 mg/l phosphinotricin and 100 ug/ml kanamycin, and resistant calli are regenerated into plants. After induction of shoots and roots, which proves normal growth of the plants despite the presence of the RNase T1 gene, the transformants are transferred to the greenhouse and are grown until they flower. The flowers are examined and show no normal style-stigma formation. After pollination, no viable seeds are formed. The transformed plants are female-sterile.

### Example 6 - Construction of chimaeric DNA sequences of a PSTMG and a Barnase gene

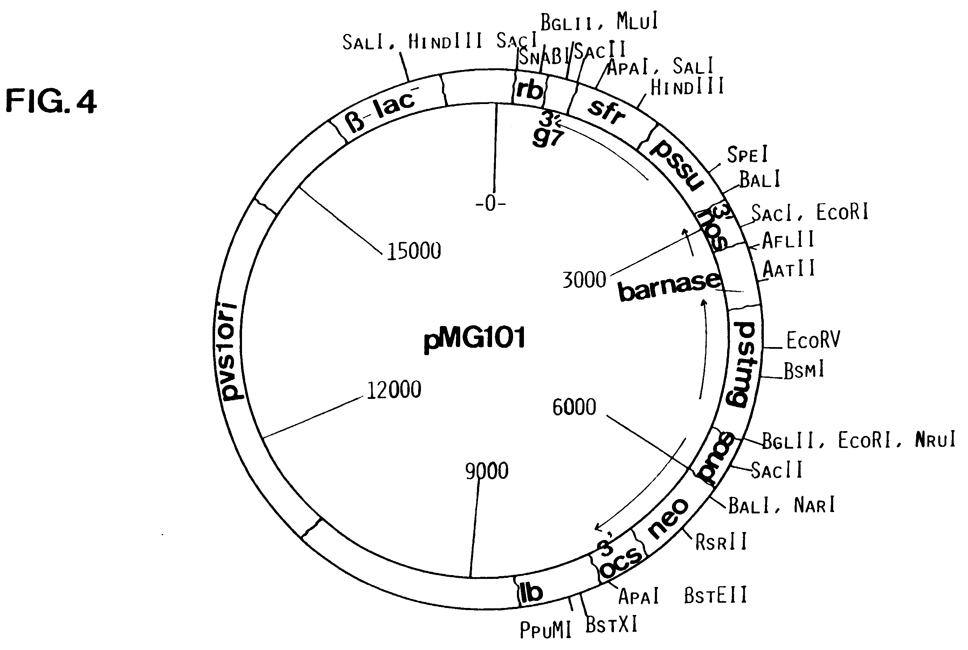

Plasmids named "pmg101", shown in Fig.4, are constructed, each by assembling the following well known DNA fragments with a different one of the PSTMGs of Example 3:
1. a vector fragment, including T-DNA border sequences, derived from pGSC1700 as described in Example 4 and with the following DNA fragments 2-4 between its border sequences;
2. the chimaeric sequence (no. 2) of Example 4, containing the PSSU promoter, the herbicide-resistance gene sfr and the 3′ end of T-DNA gene 7;
3. the chimaeric sequence (no. 3) of Example 4, containing the PNOS promoter, the neo gene encoding kanamycin resistance and the 3′ end signals of the OCS gene; and
4. a chimaeric sequence, containing one of the PSTMGs from Example 3, fused in frame with the Barnase gene from Bacillus amiloliquefaciens (Hartley and Rogerson (1972) Preparative Biochemistry 2 (3), 243-250) and the 3′ end of the NOS gene of Example 4.

Each pMG101 is a binary type T-DNA vector containing, within the T-DNA border sequences, three chimaeric sequences: PSSU-sfr and PNOS-neo which are markers DNAs with respectively PSSU and PNOS as second promoters; and PSTMG-Barnase gene which is a female-sterility DNA under the control of a PSTMG as a first promoter. Expression of the female-sterility DNA under the control of the PSTMG promoter will produce Barnase selectively in style and/or stigma cells. This will be lethal for the style and/or stigma cells since Barnase will degrade the RNA molecules and thereby interfere with the metabolism of these cells.

### Example 7 - Introduction of each chimaeric DNA sequence of Example 6 into tobacco and alfalfa

As described in Example 5, a recombinant Agrobacterium strain is constructed by mobilizing each pMG101 (from Example 6) from E. coli into Agrobacterium C58C1 Rif^{R} containing pMP90 (Koncz and Schell (1986) Mol. Gen. Genetics 204, 383-396). The resulting strain, harboring pMP90 and pMG101, is used for tobacco leaf disc transformation and for alfalfa transformation. Transformed calli and shoots are selected using 5mg/l phosphinothricin and 100 ug/ml kanamycin. That the Barnase gene is not expressed in the transformed herbicide-resistant calli and shoots is shown by their growth.

The transformed shoots are rooted, transferred to soil in the greenhouse and grown until they flower. The flowers of both the tobacco and alfalfa are examined, and essentially the same phenotype is observed in the transformed plants as is observed in the transformed plants described in Example 5 (i.e., no normal style-stigma formation). The transformed plants are female-sterile.

### Example 8 - Construction of chimaeric DNA sequences of a PSTMG and a gene encoding papain

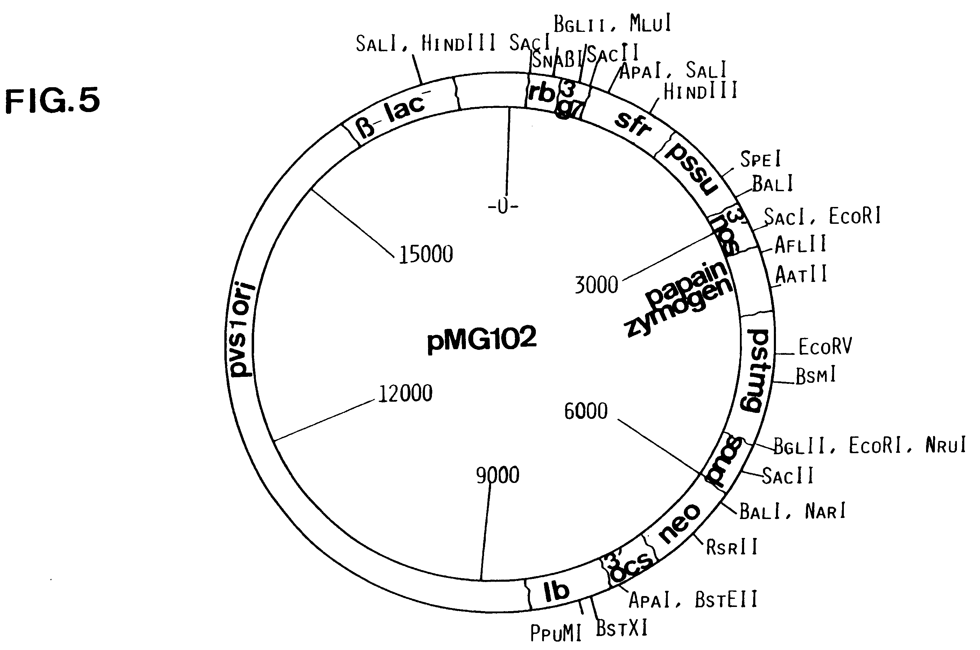

Plasmids named "pMG102", shown in Fig 5, are constructed, each by assembling the following well known DNA fragments with a different one of the PSTMGs of Example 3:
1. a vector fragment, including T-DNA border sequences, derived from pGSC1700 as described in Example 4 and with the following DNA fragments 2-4 between its border sequences;
2. the chimaeric sequence (no. 2) of Example 4, containing the PSSU promoter, the herbicide resistance gene sfr and the 3′ end of T-DNA gene 7.
3. the chimaeric sequence (no. 3) of Example 4, containing the PNOS promoter, the neo gene and the 3′ end of the OCS gene; and
4. a chimaeric sequence, containing one of the PSTMGs from Example 3, fused in frame with:
   a) a papain gene from Carica papaya fruit, encoding the papain zymogen which is a plant endopeptidase (Cohen et al (1986) Gene 48, 219-227) capable of attacking peptide, as well as ester, bonds; the following modifications are made in the DNA sequence of the papain gene according to Cohen et al (1986), using site directed mutagenesis as described in Example 3:
      i. the nucleotide A, position-1 upstream of the first ATG codon, is mutated into nucleotide C in order to obtain a suitable NcoI cloning site; and
      ii. the GAA codons encoding glutamate at positions 47, 118 and 135 are mutated into CAA codons encoding glutamine; and
   b) the 3′ end of the NOS gene of Example 4.

Each pMG102 is a binary type T-DNA vector containing, within the T-DNA border sequences, three chimaeric sequences: PSSU-sfr and PNOS-neo which are marker DNAs encoding dominant selectable markers for plant transformation under the control of respectively PSSU and PNOS as second promoters; and PSTMG-Papain zymogen gene which is a female-sterility DNA under the control of a PSTMG as a first promoter. Expression of the female-sterility DNA under the control of the PSTMG promoter will produce, selectively in style and/or stigma cells, an endopeptidase (the papain zymogen) that will cleave proteins in the style and/or stigma cells, thus leading to the death of these cells.

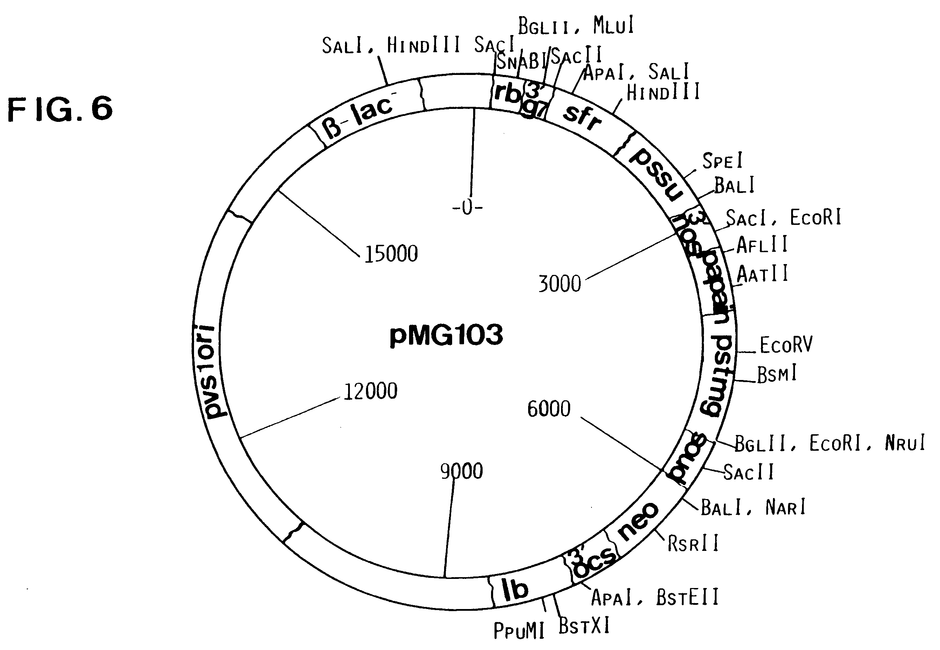

Plasmids named "pMG103", shown in Fig.6, are also constructed, each by assembling the following well known DNA fragments with a different one of the PSTMGs of Example 3:
1. a vector fragment, including T-DNA border sequences, derived from pGSC1700 as described in Example 4 and with the following DNA fragments 2-4 between its border sequences;
2. the chimaeric sequence (no. 2) of Example 4, containing the PSSU promoter, the herbicide resistance gene sfr and the 3′ end of T-DNA gene 7;
3. the chimaeric sequence (no. 3) of Example 4, containing the PNOS promoter, the neo gene, and the 3′ end of the OCS gene; and
4. a chimaeric sequence, containing one of the PSTMGs of Example 3, fused in frame with:
   a) a papain gene from Carica papaya fruit, encoding the active protein of the papain zymogen; the following modifications are made in the DNA sequence of the papain gene according to Cohen et al (1986), using site directed mutagenesis as described in Example 3:
      i. the AAT codon encoding Asn, upstream of the first Ile residue of the active protein, is mutated into a GAT codon, which provides a suitable EcoRV cloning site (GAT ATC). The EcoRV engineered site is fused directly to the PSTMG in order to obtain a direct in frame fusion of the promoter with the sequence encoding the active protein of the papain zymogen; and
      ii. the GAA codons encoding glutamate at positions 47, 118 and 135 are mutated into CAA codons encoding glutamine; and
      b) the 3′ end of the NOS gene of Example 4.

Each pMG103, like each pMG102, is a binary type T-DNA vector containing, within the T-DNA border sequences, three chimaeric genes: PSSU-sfr and PNOS-neo encoding dominant selectable markers for plant transformation; and PSTMG-Papain active protein gene which is a female-sterility DNA that is under the control of a PSTMG as a first promoter and that encodes an endopeptidase that will cleave proteins in style and/or stigma cells, thus leading selectively to the death of these cells.

### Example 9 - Introduction of each chimaeric DNA sequence of Example 8 into tobacco and alfalfa

As described in Example 5, each pMG102 and pMG103 (from Example 8) is mobilized from E. coli into separate Agrobacteria C58C1 Rif^{R} carrying pMP90. The resulting strains, harboring pMP90 with pMG102 and pMP90 with pMG103, are used to transform tobacco and alfalfa following the procedures of Example 5. That the papain genes are not expressed in transformed herbicide- and kanamycin-resistant calli, shoots and roots is shown by their growth.

The transformed plants are transferred into the greenhouse and grown in soil until they flower. The flowers of both the tobacco and alfalfa are examined, and essentially the same phenotypes are observed in the transformed plants as are observed in the transformed plants described in Example 5 (i.e., no normal style-stigma formation). The transformed plants are female-sterile.

### Example 10 - Construction of chimaeric DNA sequences of a PSTMG and a gene encoding EcoRI

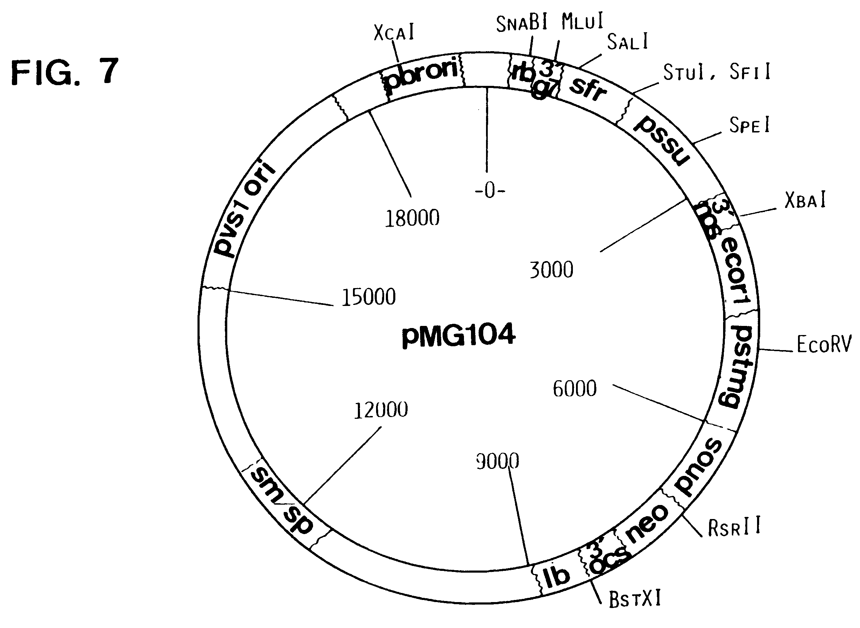

Plasmids named "pMG104", shown in Fig. 7, are constructed, each by assembling the following well known DNA fragments with a different one of the PSTMGs of Example 3:
1. a vector fragment, including T-DNA border sequences, derived from pGSC1701A2 (European patent application 87/115985.1); located between the border sequences are the following DNA fragments 2-5;
2. the chimaeric sequence (no. 2) of Example 4, containing the PSSU promoter, the herbicide-resistance gene sfr and the 3′ end of T-DNA gene 7;
3. the chimaeric sequence (no. 3) of Example 4, containing the PNOS promoter, the neo gene and the 3′ end of the OCS gene;
4. a chimaeric sequence, containing one of the PSTMGs of Example 3, fused in frame with:
   a) a gene encoding the EcoRI restriction endonuclease from an E. coli (Green et al (1981) J. Biol. Chem. 256, 2143-2153; Botterman and Zabeau (1985) Gene 37, 229-239) and capable of recognizing and cleaving the target sequence GAATTC on a double stranded DNA; the following modifications are made in the DNA sequence of the gene according to Green et al (1981) using site directed mutagenesis as described in Example 3:
      i. the nucleotides of the ATG initiation codon are replaced by ATGCA, creating a NsiI site at the initiation codon and yielding the following nucleotide sequences: ATGCA,TCT,AAT...; and
      ii. the HindII-HindIII fragment of the EcoRI gene cloned in pEcoR12 (Botterman and Zabeau, 1985) is cloned into the pMAC5-8 site directed mutagenesis vector; and
   b) the 3′ end of the NOS gene of Example 4; and
5. a gene encoding an EcoRI methylase, under the control of its natural promoter (Botterman and Zabeau, 1985), which is capable of inhibiting the activity of EcoRI in E. coli or Agrobacterium, in order to overcome potential leaky expression of the EcoRI gene in microorganisms.

Each pMG104 is a binary type T-DNA vector containing, within the T-DNA border sequences, three chimaeric sequences: PSSU-sfr and PNOS-neo which are marker DNAs under the control of respectively PSSU and PNOS as second promoters; and PSTMG-EcoRI endonuclease gene which is a female-sterility DNA under the control of a PSTMG as a first promoter. Expression of the female-sterility DNA under the control of the PSTMG promoter selectively in style and/or stigma cells will produce the EcoRI restriction endonuclease which will cleave double stranded DNA at GAATTC sites (see for review of type II restriction modification systems: Wilson (1988) TIG 4 (11), 314-318) in the style and/or stigma cells, thus leading to the death of these cells.

Plasmids named "pMG105" are also constructed, each by assembling the following well known DNA fragments with a different one of the PSTMGs of Example 3:
1. a vector fragment, including T-DNA border sequences, derived from pGSC1701A2; located between the border sequences are the following DNA fragments 2-5;
2. the chimaeric sequence (no. 2) of Example 4, containing the PSSU promoter, the herbicide-resistance gene sfr and the 3′ end of T-DNA gene 7;
3. the chimaeric sequence (no. 3) of Example 4, containing the PNOS promoter, the neo gene and the neo 3′ end of the OCS gene;
4. a chimaeric sequence, containing one of the PSTMGs of Example 3, fused in frame with:
   a) a gene fragment encoding the transit peptide of the Mn-superoxide dismutase ("Mn-SOD") which is a NcoI-PstI fragment of a HpaI-HindIII fragment from pSOD1 (Bowler et al (1989) Embo J. 8, 31-38); the following modifications are made in the DNA sequence of the gene fragment according to Bowler et al (1989) using site directed mutagenesis as described in Example 3:
      i. the AA nucleotides located upstream at position -2 and -1 of the ATG initiation codon are changed to CC nucleotides creating a NcoI site at the initiation codon and yielding the following nucleotide sequences: 
- CCATGGCACTAC
NcoI
      ii. the T,TCG,CTC nucleotides located immediately downstream of the processing site of the transit peptide are changed to C,TGC,AGC, creating a PstI site behind the processing site and yielding the following nucleotide sequences: 
L Q T F S L
CTC,CGC,GGC, TTG,CAG,ACC,TTT,TCG,CTC
CTC,CGC,GGC, TTG,CAG,ACC,TTC,TGC,AGC
PstI
 in which the arrow indicates the processing site of the transit peptide sequence and the upper line the aminoacid sequence corresponding with the Mn-SOD coding sequence; the NcoI-PstI fragment is also fused in frame with a gene encoding the EcoRI restriction endonuclease from E. coli (Greene et al (1981) J. Biol. Chem. 256, 2143-2153; Botterman and Zabeau (1985) Gene 37, 229-239) and capable of recognition and cleavage of the target sequence GAATTC on a double stranded DNA, as found in pMG104; and
   b) the 3' end of the NOS gene of Example 4; and
5. a gene encoding the EcoRI methylase under the control of its natural promoter (Botterman and Zabeau, 1985), which is capable of inhibiting the activity of EcoRI in E. coli or Agrobacterium, in order to overcome potential leaky expression of the EcoRI gene in microorganisms, this gene being inserted into the vector fragment outside the border sequences.

Each pMG105 is a binary type T-DNA vector containing, within the border sequences, three chimeric sequences: PSSU-sfr and PNOS-NPTII which are marker DNAs under the control of respectively PSSU and PNOS as second promoters; and PSTMG-transit peptide-EcoRI endonuclease gene which is a female-sterility DNA having the PSTMG as a first promoter and a transit peptide-encoding sequence between them. Expression of the female-sterility DNA under the control of the PSTMG promoter selectively in style and/or stigma cells will produce a restriction endonuclease which will be targeted into the mitochondria of the style and/or stigma cells and cleave the double stranded DNA at the GAATTC sites in such cells. This will lead to the death of these cells.

### Example 11 - Introduction of each chimaeric DNA sequence of Example 10 into tobacco and alfalfa

As described in Example 5, each pMG104 and pMG105 (from Example 10) is mobilized from E. coli into separate Agrobacteria C58C1 Rif^{R} carrying pMP90. The resulting strains, harboring pMG104 with pMP90 and pMG105 with pMP90, are used to transform tobacco and alfalfa following the procedures described in Examples 5. That the EcoRI endonuclease genes are not expressed in transformed herbicide- and kanamycin-resistant calli, shoots and roots is shown by their growth.

The transformed plants are transferred into the greenhouse and grown in soil until they flower. The flowers of both the tobacco and alfalfa are examined, and essentially the same phenotypes are observed for the transformed plants as are observed in the transformed plants described in Example 5 (i.e., no normal style-stigma formation). The transformed plants are female-sterile.

## Claims

1. A DNA which contains a first chimeric DNA which comprises:
(a) a female sterility DNA encoding a first RNA, protein or polypeptide, capable, when produced in cells of a female reproductive organ of said plant, of disturbing significantly the metabolism, functioning and/or development of the female reproductive organ; and
(b) a first promoter capable of directing expression of this DNA selectively in cells of the female reproductive organs of said plant, said female sterility DNA being in the same transcriptional unit as, and under the control of said first promoter.

2. The DNA of claim 1 in which said first promoter is capable of directing expression of said female sterility DNA especially in style cells, stigma cells, ovary cells, ovule cells and/or septum cells.

3. The DNA of claim 1, wherein said first promoter is capable of directing expression of said female-sterility DNA especially in style and/or stigma cells.

4. The DNA of any one of claims 1 to 3 wherein said first promoter is selected from the group consisting of : PSTMG07 which is the promoter of an endogenous tobacco gene encoding an mRNA from which the cDNA of Fig. 1A can be prepared; PSTMG08 which is the promoter of an endogenous tobacco gene encoding an mRNA from which the cDNA of Fig. 1B can be prepared; PSTMG4B12 which is the promoter of an endogenous tobacco gene encoding an mRNA from which the cDNA of Fig. 2A can be prepared; PSTMG3C9 which is the promoter of an endogenous tobacco gene encoding an mRNA from which the cDNA of Fig. 2B can be prepared.

5. The DNA of any one of claims 1 to 4 wherein said first promoter is a promoter of a gene corresponding to cDNA clone pMON9608.

6. The DNA of any one of claims 1 to 5 wherein said female-sterility DNA encodes a ribonuclease, particularly RNase T1.

7. The DNA of any one of claims 1 to 5 wherein said female-sterility DNA encodes a Barnase

8. The DNA of any one of claims 1 to 5 wherein said female-sterility DNA encodes a DNase, especially an endonuclease, particularly EcoRI; a protease especially a papain, particularly papain Zymogen or papain active protein; a glucanase: a lipase, particularly phospholipase A₂; a lipid peroxidase; a cell wall inhibitor; a bacterial toxin; or a ribozyme; particularly a ribozyme against mRNA encoded by any of the STMG-type genes, or by a gene corresponding to cDNA clone pMON9608 ; or alternatively, wherein said female-sterility DNA: encodes a ribonuclease, particularly T₂ or Rh: encodes a glycoprotein encoded by the S1, S2, S3, S6 or S7 alleles, particularly of Nicotiana alata; or is an anti-sense DNA, particularly an anti-sense DNA encoding an RNA which is complementary to the mRNA of an STMG-type gene.

9. The DNA of any one of claims 1 to 5, wherein said female-sterility DNA encodes an enzyme which catalyzes the synthesis of a phytohormone, particularly the enzyme encoded by gene 4 of Aprobacterium T-DNA, or an enzyme encoded by gene 1 and/or gene 2 of Aprobacterium T-DNA.

10. The DNA of any one of claims 1 to 5 wherein said female-sterility DNA encodes a viral dependant RNA polymerase, particularly a TNV replicase, under the control of said first promoter; and a negative strand of a gene encoding said first protein or polypeptide; said gene being in the same transcriptional unit as, and under the control of, a promoter, such as another first promoter, capable of directing expression of said negative strand in the cell; said gene also being fused at its 3' end to a viral subgenomic promoter, such as the TNV subgenomic promoter, which, is specifically recognized by said viral dependent RNA polymerase.

11. The DNA of any one of clams 1 to 10 wherein said DNA further comprises:
(e) a first DNA encoding a transit peptide capable of transporting said first protein or polypeptide into a chloroplast or mitochondria of said female reproductive organ cells: said first DNA being in the same transcriptional unit as said female-sterility DNA and said first promoter and between said female-sterility DNA and said first promoter.

12. The DNA of any one of claims 1 to 10 wherein said DNA further comprises:
(e) a third DNA encoding a secretory signal peptide capable of secreting said first protein or polypeptide outside the cell of a female reproductive organ of a plant; said third DNA being in the same transcriptional unit as said female-sterility DNA and said first promoter and located between said female-sterility DNA and said first promoter.

13. The DNA of anyone of claims 1 to 12 which also comprises a second chimeric DNA, adjacent to said first chimeric DNA, said second chimeric DNA comprising:
(c) a marker DNA encoding a second RNA, protein or polypeptide which, when present at least in a specific tissue or in at least specific cells of a plant, renders said plant easily separable from other plants which do not contain said second RNA, protein or polypeptide in said specific tissue or specific cells; and
(d) a second promoter capable of directing expression of said marker DNA at least in said specific tissue or specific cells: said marker DNA being in the same transcriptional unit as, and under the control of, said second promoter.

14. The DNA of claims 13 wherein said marker DNA encodes a protein inhibiting or neutralizing the action of a herbicide.

15. The DNA of claim 14 in which said marker DNA is an herbicide resistance gene, particularly a gene conferring resistance to a glutamine synthetase inhibitor, such as phosphinothricin.

16. The DNA of claim 15 in which said marker DNA is a sfr or sfrv gene.

17. The DNA of claim 14 wherein said marker DNA is a gene encoding a modified target enzyme for an herbicide having tower affinity for the herbicide, particularly a modified 5-enolpyruvylshikimate-3 phosphate synthase as a target for glyphosate or a modified glutamine synthetase as a target for a glutamine synthetase inhibitor such as phosphinotricin.

18. The DNA of claim 13, wherein said marker DNA is a gene encoding a protein or a polypeptide conferring a color to at least said specific tissue or specific cells, particularly the gene A1 or the GUS gene; a gene encoding a protein or a polypeptide conferring a stress tolerance to said plant, particularly the gene encoding Mn-superoxide dismutase; or a gene encoding a protein or a polypeptide conferring a disease or pest resistance, particularly a gene encoding a *Bacillus thuringiensis* endotoxin that confers insect resistance or a gene encoding a bactericidal peptide that confers a bacterial resistance.

19. The DNA of any one of claims 13 to 18 wherein said second promoter is: a constitutive promoter, particularly a PNOS promoter or a POCS promoter; a wound-inducible promoter, particularly a TR1' or TR2' promoter; a promoter which directs gene expression selectively in plant tissue having photosynthetic activity, or a promoter which directs gene expression selectively in leaf cells, petal cells or seed cells, particularly seed coat cells.

20. The DNA of claim 19 wherein said second promoter is a 35S promoter or an SSU promoter.

21. The DNA of any one of claims 13 to 20 wherein said DNA further comprises:
(h) a second DNA encoding a transit peptide capable of transporting said second protein or polypeptide into a chloroplast or mitochondria of at least said specific tissue or specific cells; said second DNA being in the same transcriptional unit as said marker DNA and said second promoter and between said marker DNA and said second promoter.

22. The DNA of any one of claims 13 to 20 wherein said DNA further comprises:
(f) a fourth DNA encoding a secretory signal peptide capable of secreting said second protein or polypeptide outside at least specific tissue or specific cells; said fourth DNA being in the same transcriptional unit as said marker DNA and said second promoter and located between said marker DNA and said second promoter.

23. A DNA which comprises the T-DNA of pMG100 of Fig. 3, pMG101 of Fig.4, pMG102 of Fig. 5, pMG103 of Fig. 6, pMG104 of Fig. 7, or pMG105 of Fig. 8.

24. The DNA of any one of claims 1 to 23 which is nuclear DNA of a cell of a plant or of a seed.

25. A vector which contains the DNA of any one of claims 1 to 23.

26. A cell of a plant which contains the DNA of any one of claims 1 to 24.

27. A cell of a plant which contains, stably integrated into its nuclear DNA, the DNA of any one of claims 1 to 23.

28. A cell of claim 26 or claim 27 which can be regenerated into a plant which is female-sterile.

29. A plant cell culture containing plant cells of any one of claims 26 to 28.

30. A plant which contains the DNA of any one of claims 1 to 24.

31. A plant which contains the DNA of any one of claims 1 to 24 in all of its cells.

32. The plant of claim 30 or 31 which is female-sterile.

33. The plant of any one of claims 30 to 32 which is a hybrid plant.

34. A female-sterile plant containing a foreign DNA incorporated in the nuclear genome of its cells, wherein said foreign DNA comprises :
(a) a female-sterility DNA encoding a first RNA, protein or polypeptide, capable when produced in cells of a female reproductive organ of said plant, of disturbing significantly the metabolism, functioning and/or development of the female reproductive organ; and
(b) a first promoter capable of directing expression of this DNA selectively in cells of the female reproductive organs of said plant, said female sterility DNA being in the same transcriptional unit as, and under the control of said first promoter,
provided that, if said first promoter is a promoter capable of directing expression of said female-sterility DNA selectively in female gametes, or in cells derived from female gametes, the nuclear genome of said plant is homozygous.

35. The plant of anyone of claims 30 to 34 which is selected from the group consisting of alfalfa and tobacco.

36. A plant seed which contains the DNA of any one of claims 1 to 24.

37. A seedless fruit of a plant which contains the DNA of any one of claims 1 to 24.

38. A process for producing a female-sterile plant and reproductive material for said plant, which comprises :
a) introducing the foreign DNA of any one of claims 1 to 23 into the nuclear genome of a plant cell to obtain a transformed plant cell;
b) regenerating said female-sterile plant from said transformed plant cell and; optionally,
c) obtaining said reproductive material from said female-sterile plant which contain said foreign DNA.

39. The process of claim 20 in which said reproductive material is a seed, particularly a hybrid seed, and which further comprises :
a) providing a plant which contains the DNA of any one of claims 1 to 23 incorporated in the nuclear DNA of all of its cells and which is female-sterile;
b) cross-pollinating i) said female-sterile plant, and ii) a female-fertile plant, and
c) recovering said seed from said female-fertile plant.

40. The process of claim 20 in which said reproductive material is a seed, particularly a hybrid seed, which comprises the steps of :
a) providing a plant which contains the DNA of any one of claims 13 to 23, including both said second promoter and said marker DNA, incorporated in the nuclear DNA of all of its cells, and which is female-sterile;
b) cross-pollinating i) said female-sterile plant, and ii) a female-fertile plant without said marker DNA and/or said second promoter, and
c) recovering the seed from said female-fertile plant,
wherein said process optionally comprises removing undesired female-fertile plants on the basis of the absence of expression of the marker DNA in said female-fertile plants.

41. A process for producing a seed which comprises the steps of:
(a) providing a plant which contains the DNA of any one of claims 13 to 23 incorporated in the nuclear DNA of all of its cells, which is female-sterile, and in which said marker DNA is a herbicide resistance gene, particularly a gene conferring resistance to a glutamine synthetase inhibitor such as the sfr or sfrv gene, or a gene encoding a modified target enzyme for a herbicide having lower affinity for a herbicide, particularly a modified 5-enolpyruvylshikimate-3 phosphate synthase as a target for glyphosate or a modified glutamine synthetase as a target for a glutamine synthase inhibitor such as phosphinothricin,
(b) cross-pollinating i) said female-sterile plant, and ii) a female-fertile plant without said marker DNA and/or said second promoter, and
(c) recovering the seed from said female-fertile plant,
wherein said process comprises removing undesired female-fertile plants by application of said herbicide, particularly a herbicide comprising phosphinothricin.

42. A process according to claim 41 in which said seed is a hybrid seed.

43. The process of anyone of claims 41 or 42, wherein said female-sterile plant contains in addition to said herbicide resistance gene at least another marker DNA stably integrated into the nuclear genome of its cells in the same genetic locus as said female-sterility DNA and said female-fertile plant does not contain said another marker DNA.

44. A process according to anyone of claims 39 to 43, in which the female-fertile plant is male-sterile.

45. A process according to claim 44, wherein said female-fertile male-sterile plant comprises a foreign DNA incorporated in the nuclear genome of its cells, wherein said foreign DNA comprises:
(a) a male sterility DNA encoding a third RNA, protein or polypeptide, capable when produced in stamen cells of said plant, of disturbing significantly the metabolism, functioning and/or development of the stamen cells.
(b) a third promoter capable of directing expression of this DNA selectively in stamen cells of said plant, said male sterility DNA being in the same transcriptional unit as, and under the control of said third promoter;
provided that, if said third promoter is a promoter capable of directing expression of said male-sterility DNA selectively in pollen cells, the nuclear genome of said transformed plant cell is homozygous.

46. A process according to claim 45, wherein said foreign DNA of said female-fertile male-sterile plant also comprises :
(d) a second marker DNA encoding a fourth RNA, protein or polypeptide which, when present at least in a specific tissue or in at least specific cells of said plant, renders said plant easily separable from other plants which do not contain said fourth RNA, protein or polypeptide in said specific tissue or specific cells; and
(e) a fourth promoter capable of directing expression of said second marker DNA at least in said specific tissue or specific cells; said second marker DNA being in the same transcriptional unit as, and under the control of, said fourth promoter.

47. A process according to anyone of claims 45 or 46, wherein said foreign DNA further comprises :
(f) a fifth DNA encoding a transit peptide capable of transporting said third protein or polypeptide into a chloroplast or mitochondria of said stamen cells; said fifth DNA being in the same transcriptional unit as said male-sterility DNA and said third promoter and between said male-sterility DNA and said third promoter; and/or
(g) a sixth DNA encoding a transit peptide capable of transporting said fourth protein or polypeptide into a chloroplast or mitochondria of at least said specific tissue or specific cells; said sixth DNA being in the same transcriptional unit as said second marker DNA and said fourth promoter and between said second marker DNA and said fourth promoter.

48. A pair of parent plants for producing seeds comprising: a) a female-sterile parent plant containing the DNA of any one of claims 1 to 23 incorporated in the nuclear DNA of all of its cells, and b) a female-fertile parent plant.

49. The pair of claim 48 wherein said female-sterile parent plant and said female-fertile parent plant belong to different lines.

50. The pair of claim 48 wherein said female-sterile parent plant and said female-fertile parent plant are derived from the same inbred line.

51. The pair of any one of claims 48 to 50 wherein said female-fertile parent plant is male-sterile.

## Patentansprüche

1. DNA, die eine erste chimäre DNA enthält, welche umfasst:
(a) eine DNA für weibliche Sterilität, die eine erste RNA, ein erstes Protein oder Polypeptid kodiert, die bei Erzeugung in Zellen eines weiblichen Fortpflanzungsorgans der Pflanze fähig sind, signifikant den Stoffwechsel, die Funktion und/oder die Entwicklung des weiblichen Fortpflanzungsorgans zu stören; und
(b) einen ersten Promotor, der zur selektiven Ausrichtung der Expression dieser DNA in Zellen der weiblichen Fortpflanzungsorgane der Pflanze fähig ist, wobei die DNA für weibliche Sterilität in der gleichen Transkriptionseinheit wie und unter der Kontrolle des ersten Promotors ist.

2. DNA gemäss Anspruch 1, worin der erste Promotor zur Ausrichtung der Expression der DNA für weibliche Sterilität speziell in Griffelzellen, Narbenzellen, Fruchtknotenzellen, Samenanlagenzellen und/oder Septumzellen fähig ist.

3. DNA gemäss Anspruch 1, worin der erste Promotor zur Ausrichtung der Espression der DNA für weibliche Sterilität speziell in Griffel- und/oder Narbenzellen fähig ist.

4. DNA gemäss einem der Ansprüche 1 bis 3, worin der erste Promotor ausgewählt ist aus der Gruppe, bestehend aus: PSTMG07, das der Promotor eines endogenen Tabakgens ist, das eine mRNA kodiert, aus der die cDNA der Fig. 1A hergestellt werden kann; PSTMG08, das der Promotor eines endogenen Tabakgens ist, das eine mRNA kodiert, aus der die cDNA der Fig. 1B hergestellt werden kann; PSTMG4B12, das der Promotor eines endogenen Tabakgens ist, das eine mRNA kodiert, aus der die cDNA der Fig. 2A hergestellt werden kann; PSTMG3C9, das der Promotor eines endogenen Tabakgens ist, das eine mRNA kodiert, aus der die cDNA der Fig. 2B hergestellt werden kann.

5. DNA gemäss einem der Ansprüche 1 bis 4, worin der erste Promotor ein Promotor eines Gens ist, das dem cDNA-Klon pMON9608 entspricht.

6. DNA gemäss einem der Ansprüche 1 bis 5, worin die DNA für weibliche Sterilität eine Ribonuklease kodiert, insbesondere RNase T1.

7. DNA gemäss einem der Ansprüche 1 bis 5, worin die DNA für weibliche Sterilität eine Barnase kodiert.

8. DNA gemäss einem der Ansprüche 1 bis 5, worin die DNA für weibliche Sterilität eine DNase kodiert, speziell eine Endonuklease, insbesondere EcoRI; eine Protease, speziell ein Papain, insbesondere Papain-Zymogen oder Papain-aktives Protein; eine Glucanase; eine Lipase, insbesondere Phospholipase A₂; eine Lipidperoxidase; einen Zellwandinhibitor; ein Bakteriotoxin; oder ein Ribozym; insbesondere ein Ribozym gegen mRNA, die durch eines der Gene vom STMG-Typ oder durch ein dem cDNA-Klon pMON9608 entsprechendes Gen kodiert wird; oder worin die DNA für weibliche Sterilität alternativ eine Ribonuklease kodiert, insbesondere T₂ oder Rh; ein Glycoprotein kodiert, das durch die S1-, S2-, S3-, S6- oder S7-Allele kodiert wird, insbesondere von Nicotiana alata; oder eine Antisense-DNA ist, insbesondere eine Antisense-DNA, die eine RNA kodiert, die komplementär zur mRNA eines Gens vom STMG-Typ ist.

9. DNA gemäss einem der Ansprüche 1 bis 5, worin die DNA für weibliche Sterilität eine Enzym kodiert, das die Synthese eines Phytohormons katalysiert, insbesondere das Enzym, das durch Gen 4 von Agrobacterium T-DNA kodiert wird, oder ein Enzym, das durch Gen 1 und/oder Gen 2 von Agrobacterium T-DNA kodiert wird.

10. DNA gemäss einem der Ansprüche 1 bis 5, worin die DNA für weibliche Sterilität eine virusabhängige RNA-Polymerase, insbesondere eine TNV-Replikase, unter der Kontrolle des ersten Promotors kodiert; und einen Negativstrang eines Gens, das das erste Protein oder Polypeptid kodiert; wobei das Gen in der gleichen Transkriptionseinheit wie und unter der Kontrolle eines Promotors ist, wie eines anderen ersten Promotors, der zur Ausrichtung der Expression des Negativstrangs in der Zelle fähig ist; wobei das Gen ebenfalls an seinem 3'-Ende an einen viralen subgenomischen Promotor fusioniert ist, wie den TNV-Subgenompromotor, der spezifisch durch die virusabhängige RNA-Polymerase erkannt wird.

11. DNA gemäss einem der Ansprüche 1 bis 10, worin die DNA ausserdem umfasst:
(e) eine erste DNA, die ein Transitpeptid kodiert, das zum Transport des ersten Proteins oder Polypeptids in einen Chloroplasten oder in Mitochondrien der Zellen des weiblichen Fortpflanzungsorgans fähig ist; wobei die erste DNA in der gleichen Transkriptionseinheit wie die DNA für weibliche Sterilität und der erste Promotor ist und zwischen der DNA für weibliche Sterilität und dem ersten Promotor ist.

12. DNA gemäss einem der Ansprüche 1 bis 10, worin die DNA ausserdem umfasst:
(e) eine dritte DNA, die ein Sekretionssignalpeptid kodiert, das zur Sekretion des ersten Proteins oder Polypeptids ausserhalb der Zelle eines weiblichen Fortpflanzungsorgans einer Pflanze fähig ist; wobei die dritte DNA in der gleichen Transkriptionseinheit wie die DNA für weibliche Sterilität und der erste Promotor ist und sich zwischen der DNA für weibliche Sterilität und dem ersten Promotor befindet.

13. DNA gemäss einem der Ansprüche 1 bis 12, die ebenfalls eine zweite chimäre DNA umfasst, die an die erste chimäre DNA angrenzt, wobei die zweite chimäre DNA umfasst:
(c) eine Marker-DNA, die eine zweite RNA, ein zweites Protein oder Polypeptid kodiert, welche bei Vorliegen wenigstens in einem spezifischen Gewebe oder in wenigstens spezifischen Zellen einer Pflanze die Pflanze leicht abtrennbar von anderen Pflanzen machen, die nicht die zweite RNA, das zweite Protein oder Polypeptid im spezifischen Gewebe oder in den spezifischen Zellen enthalten; und
(d) einen zweiten Promotor, der zur Ausrichtung der Expression der Marker-DNA wenigstens in dem spezifischen Gewebe oder in den spezifischen Zellen fähig ist; wobei die Marker-DNA in der gleichen Transkriptionseinheit wie und unter der Kontrolle des zweiten Promotors ist.

14. DNA gemäss Anspruch 13, worin die Marker-DNA ein Protein kodiert, das die Wirkung eines Herbizids hemmt oder neutralisiert.

15. DNA gemäss Anspruch 14, worin die Marker-DNA ein Herbizidresistenz-Gen ist, insbesondere ein Gen, das Resistenz gegen einen Glutaminsynthetase-Inhibitor wie Phosphinothricin verleiht.

16. DNA gemäss Anspruch 15, worin die Marker-DNA ein sfr- oder sfrv-Gen ist.

17. DNA gemäss Anspruch 14, worin die Marker-DNA ein Gen ist, das ein modifiziertes Zielenzym für ein Herbizid mit geringerer Affinität für das Herbizid kodiert, insbesondere eine modifizierte 5-Enolpyruvylshikimat-3-phosphatsynthetase als Ziel für Glyphosat oder eine modifizierte Glutaminsynthetase als Ziel für einen Glutaminsynthetase-Inhibitor wie Phosphinotricin.

18. DNA gemäss Anspruch 13, worin die Marker-DNA ein Gen ist, das ein Protein oder Polypeptid kodiert, das wenigstens dem spezifischen Gewebe oder den spezifischen Zellen eine Färbung verleiht, insbesondere das Gen A1 oder das GUS-Gen; ein Gen, das ein Protein oder Polypeptid kodiert, das der Pflanze Stresstoleranz verleiht, insbesondere das Gen, das Mn-Superoxiddismutase kodiert; oder ein Gen, das ein Protein oder Polypeptid kodiert, das eine Krankheits- oder Schädlingsresistenz verleiht, insbesondere ein Gen, das ein Bacillus thuringiensis-Endotoxin kodiert, welches Insektenresistenz verleiht, oder ein Gen, das ein bakterizides Peptid kodiert, das Bakterienresistenz verleiht.

19. DNA gemäss einem der Ansprüche 13 bis 18, worin der zweite Promotor ein konstitutiver Promotor, insbesondere ein PNOS-Promotor oder ein POCS-Promotor ist; ein wundeninduzierbarer Promotor, insbesondere ein TR1'- oder TR2'-Promotor; ein Promotor, der die Genexpression selektiv in Pflanzengewebe mit Photosyntheseaktivität ausrichtet, oder ein Promotor, der die Genexpression selektiv in Blattzellen, Blütenblattzellen oder Samenzellen ausrichtet, insbesondere Samenschalenzellen.

20. DNA gemäss Anspruch 19, worin der zweite Promotor ein 35S-Promotor oder ein SSU-Promotor ist.

21. DNA gemäss einem der Ansprüche 13 bis 20, worin die DNA ausserdem umfasst:
(h) eine zweite DNA, die ein Transitpeptid kodiert, das zum Transport des zweiten Proteins oder Polypeptids in einen Chloroplasten oder in Mitochondrien wenigstens des spezifischen Gewebes oder der spezifischen Zellen fähig ist; wobei die zweite DNA in der gleichen Transkriptionseinheit wie die Marker-DNA und der zweite Promotor ist und zwischen der Marker-DNA und dem zweiten Promotor ist.

22. DNA gemäss einem der Ansprüche 13 bis 20, worin die DNA ausserdem umfasst:
(f) eine vierte DNA, die ein Sekretionssignalpeptid kodiert, das zur Sekretion des zweiten Proteins oder Polypeptids ausserhalb wenigstens des spezifischen Gewebes oder spezifischer Zellen fähig ist; wobei die vierte DNA in der gleichen Transkriptionseinheit wie die Marker-DNA und der zweite Promotor ist und sich zwischen der Marker-DNA und dem zweiten Promotor befindet.

23. DNA, die die T-DNA von pMG100 aus Fig. 3, pMG101 aus Fig. 4, pMG102 aus Fig. 5, pMG103 aus Fig. 6, pMG104 aus Fig. 7 oder pMG105 aus Fig. 8 umfasst.

24. DNA gemäss einem der Ansprüche 1 bis 23, die eine Zellkern-DNA einer Zelle einer Pflanze oder eines Samens ist.

25. Vektor, der die DNA gemäss einem der Ansprüche 1 bis 23 enthält.

26. Zelle einer Pflanze, die die DNA gemäss einem der Ansprüche 1 bis 24 enthält.

27. Zelle einer Pflanze, die stabil in ihre Zellkern-DNA integriert die DNA gemäss einem der Ansprüche 1 bis 23 enthält.

28. Zelle gemäss Anspruch 26 oder 27, die zu einer Pflanze regeneriert werden kann, die weiblich-steril ist.

29. Pflanzenzellkultur, die Pflanzenzellen gemäss einem der Ansprüche 26 bis 28 enthält.

30. Pflanze, die die DNA gemäss einem der Ansprüche 1 bis 24 enthält.

31. Pflanze, die die DNA gemäss einem der Ansprüche 1 bis 24 in allen ihren Zellen enthält.

32. Pflanze gemäss Anspruch 30 oder 31, die weiblichsteril ist.

33. Pflanze gemäss einem der Ansprüche 30 bis 32, die eine Hybridpflanze ist.

34. Weibliche-sterile Pflanze, die eine in das Kerngenom ihrer Zellen aufgenommene Fremd-DNA enthält, worin die Fremd-DNA umfasst:
(a) eine DNA für weibliche Sterilität, die eine erste RNA, ein erstes Protein oder Polypeptid kodiert, die bei Erzeugung in Zellen eines weiblichen Fortpflanzungsorgans der Pflanze zur signifikanten Störung des Stoffwechsels, der Funktion und/oder der Entwicklung des weiblichen Fortpflanzungsorgans fähig sind; und
(b) einen ersten Promotor, der zur selektiven Ausrichtung der Expression dieser DNA in Zellen der weiblichen Fortpflanzungsorgane der Pflanze fähig ist, wobei die DNA für weibliche Sterilität in der gleichen Transkriptionseinheit wie und unter der Kontrolle des ersten Promotors ist,
mit der Massgabe, dass das Kerngenom der Pflanze homozygot ist, falls der erste Promotor ein Promotor ist, der zur selektiven Ausrichtung der Expression der DNA für weibliche Sterilität in weiblichen Gameten oder in aus weiblichen Gameten stammenden Zellen fähig ist.

35. Pflanze gemäss einem der Ansprüche 30 bis 34, die ausgewählt ist aus der Gruppe bestehend aus Luzerne und Tabak.

36. Pflanzensamen, der die DNA gemäss einem der Ansprüche 1 bis 24 enthält.

37. Samenlose Frucht einer Pflanze, die die DNA gemäss einem der Ansprüche 1 bis 24 enthält.

38. Verfahren zur Erzeugung einer weiblich-sterilen Pflanze und von Fortpflanzungsmaterial für die Pflanze, welches umfasst:
(a) Einführen der Fremd-DNA gemäss einem der Ansprüche 1 bis 23 in das Kerngenom einer Pflanzenzelle zum Erhalt einer transformierten Pflanzenzelle;
(b) Regenerieren der weiblich-sterilen Pflanze aus der transformierten Pflanzenzelle und gegebenenfalls
(c) Erhalten des Fortpflanzungsmaterials aus der weiblich-sterilen Pflanze, das die Fremd-DNA enthält.

39. Verfahren gemäss Anspruch 20, worin das Fortpflanzungsmaterial ein Samen ist, insbesondere ein Hybridsamen, und welches ausserdem umfasst:
(a) Bereitstellen einer Pflanze, die die DNA gemäss einem der Ansprüche 1 bis 23 aufgenommen in die Zellkern-DNA aller ihrer Zellen enthält, und die weiblich-steril ist;
(b) Fremdbestäuben (i) der weiblich-sterilen Pflanze und (ii) einer weiblich-fruchtbaren Pflanze und
(c) Gewinnen des Samens aus der weiblichfruchtbaren Pflanze.

40. Verfahren gemäss Anspruch 20, worin das Fortpflanzungsmaterial ein Samen ist, insbesondere ein Hybridsamen, welches die Schritte umfasst:
(a) Bereitstellen einer Pflanze, die die DNA gemäss einem der Ansprüche 13 bis 23 enthält, einschliesslich sowohl des zweiten Promotors als auch der Marker-DNA, aufgenommen in die Zellkern-DNA aller ihrer Zellen, und die weiblich-steril ist;
(b) Fremdbestäuben (i) der weiblich-sterilen Pflanze und (ii) einer weiblich-fruchtbaren Pflanze ohne die Marker-DNA und/oder den zweiten Promotor, und
(c) Gewinnen des Samens aus der weiblichfruchtbaren Pflanze,
worin das Verfahren gegebenenfalls die Entfernung ungewünschter weiblich-fruchtbarer Pflanzen auf der Grundlage des Fehlens der Expression der Marker-DNA in den weiblich-fruchtbaren Pflanzen umfasst.

41. Verfahren zur Erzeugung eines Samens, das die Schritte umfasst aus:
(a) Bereitstellen einer Pflanze, die die DNA gemäss einem der Ansprüche 13 bis 23 aufgenommen in die Zellkern-DNA aller ihrer Zellen enthält, die weiblich-steril ist, und worin die Marker-DNA ein Herbizidresistenzgen ist, insbesondere ein Gen, das Resistenz gegen einen Glutaminsynthetase-Inhibitor verleiht, wie das sfr- oder sfrv-Gen, oder ein Gen, das ein modifiziertes Zielenzym für ein Herbizid mit geringerer Affinität für ein Herbizid kodiert, insbesondere eine modifizierte 5-Enolpyruvylshikimate-3-phosphatsynthetase als Ziel für Glyphosat oder eine modifizierte Glutaminsynthetase als Ziel für einen Glutaminsynthetase-Inhibitor wie Phosphinothricin,
(b) Fremdbestäuben (i) der weiblich-sterilen Pflanze und (ii) einer weiblich-fruchtbaren Pflanze ohne die Marker-DNA und/oder den zweiten Promotor, und
(c) Gewinnen des Samens aus der weiblichfruchtbaren Pflanze,
worin das Verfahren die Entfernung ungewünschter weiblich-fruchtbarer Pflanzen durch Anwendung des Herbizids umfasst, insbesondere eines Herbizids, das Phosphinothricin umfasst.

42. Verfahren gemäss Anspruch 41, worin der Samen ein Hybridsamen ist.

43. Verfahren gemäss einem der Ansprüche 41 oder 42, worin die weiblich-sterile Pflanze zusätzlich zum Herbizidresistenzgen wenigstens eine andere, stabil in das Kerngenom ihrer Zellen integrierte Marker-DNA im gleichen genetischen Locus wie die DNA für weibliche Sterilität enthält und die weiblichfruchtbare Pflanze die andere Marker-DNA nicht enthält.

44. Verfahren gemäss einem der Ansprüche 39 bis 43, worin die weiblich-fruchtbare Pflanze männlich-steril ist.

45. Verfahren gemäss Anspruch 44, worin die weiblichfruchtbare, männlich-sterile Pflanze eine in das Kerngenom ihrer Zellen aufgenommene Fremd-DNA umfasst, worin die Fremd-DNA umfasst:
(a) eine DNA für männliche Sterilität, die eine dritte RNA, ein drittes Protein oder Polypeptid kodiert, welche bei Erzeugung in Staubblattzellen der Pflanze zur signifikanten Störung des Stoffwechsels, der Funktion und/oder der Entwicklung der Staubblattzellen fähig sind;
(b) einen dritten Promotor, der zur selektiven Ausrichtung der Expression dieser DNA in Staubblattzellen der Pflanze fähig ist, wobei die DNA für männliche Sterilität in der gleichen Transkriptionseinheit wie und unter der Kontrolle des dritten Promotors ist;
mit der Massgabe, dass das Kerngenom der transformierten Pflanzenzelle homozygot ist, falls der dritte Promotor ein Promotor ist, der zur selektiven Ausrichtung der Espression der DNA für männliche Sterilität in Pollenzellen fähig ist.

46. Verfahren gemäss Anspruch 45, worin die Fremd-DNA der weiblich-fruchtbaren, männlich-sterilen Pflanze ebenfalls umfasst:
(d) eine zweite Marker-DNA, die eine vierte RNA, ein viertes Protein oder Polypeptid kodiert, welche bei Vorliegen wenigstens in einem spezifischen Gewebe oder in wenigstens spezifischen Zellen der Pflanze die Pflanze leicht abtrennbar von anderen Pflanzen machen, die die vierte RNA, das vierte Protein oder Polypeptid im spezifischen Gewebe oder in den spezifischen Zellen nicht enthalten; und
(e) einen vierten Promotor, der zur Ausrichtung der Expression der zweiten Marker-DNA wenigstens im spezifischen Gewebe oder in den spezifischen Zellen fähig ist; wobei die zweite Marker-DNA in der gleichen Transkriptionseinheit wie und unter der Kontrolle des vierten Promotors ist.

47. Verfahren gemäss einem der Ansprüche 45 oder 46, worin die Fremd-DNA ausserdem umfasst:
(f) eine fünfte DNA, die ein Transitpeptid kodiert, das zum Transport des dritten Proteins oder Polypeptids in einen Chloroplasten oder in Mitochondrien der Staubblattzellen fähig ist; wobei die fünfte DNA in der gleichen Transkriptionseinheit wie die DNA für männliche Sterilität und der dritte Promotor ist und zwischen der DNA für männliche Sterilität und dem dritten Promotor ist; und/oder
(g) eine sechste DNA, die ein Transitpeptid kodiert, das zum Transport des vierten Proteins oder Polypeptids in einen Chloroplasten oder in Mitochondrien wenigstens des spezifischen Gewebes oder der spezifischen Zellen fähig ist; wobei die sechste DNA in der gleichen Transkriptionseinheit wie die zweite Marker-DNA und der vierte Promotor ist und zwischen der zweiten Marker-DNA und dem vierten Promotor ist.

48. Paar von Mutterpflanzen zur Erzeugung von Samen, umfassend:
(a) eine weiblich-sterile Mutterpflanze, die die DNA gemäss einem der Ansprüche 1 bis 23 aufgenommen in die Zellkern-DNA aller ihrer Zellen enthält, und
(b) eine weiblich-fruchtbare Mutterpflanze.

49. Paar gemäss Anspruch 48, worin die weiblich-sterile Mutterpflanze und die weiblich-fruchtbare Mutterpflanze zu unterschiedlichen Linien gehören.

50. Paar gemäss Anspruch 48, worin die weiblich-sterile Mutterpflanze und die weiblich-fruchtbare Mutterpflanze aus der gleichen Inzuchtlinie stammen.

51. Paar gemäss einem der Ansprüche 48 bis 50, worin die weiblich-fruchtbare Mutterpflanze männlich-steril ist.

## Revendications

1. ADN qui contient un premier ADN chimérique qui comprend :
(a) un ADN de stérilité femelle codant pour un premier ARN, une protéine ou un polypeptide, capable, quand il est produit dans des cellules d'un organe de reproduction femelle de ladite plante de perturber significativement le métabolisme, le fonctionnement et/ou le développement de l'organe de reproduction femelle ; et
(b) un premier promoteur capable de diriger l'expression de cet ADN sélectivement dans des cellules des organes de reproduction femelle de ladite plante, ledit ADN de stérilité femelle étant dans la même unité transcriptionnelle que, et sous la régulation dudit premier promoteur.

2. ADN selon la revendication 1, dans lequel ledit premier promoteur est capable de diriger l'expression dudit ADN de stérilité femelle en particulier dans des cellules de style, des cellules de stigmate, des cellules d'ovaire, des cellules d'ovule et/ou des cellules de septum.

3. ADN selon la revendication 1, dans lequel ledit premier promoteur est capable de diriger l'expression dudit ADN de stérilité femelle en particulier dans des cellules de style et/ou de stigmate.

4. ADN selon l'une quelconque des revendications 1 à 3, dans lequel ledit premier promoteur est choisi dans le groupe constitué de : PSTMG07 qui est le promoteur d'un gène endogène de tabac codant pour un ARNm d'où l'ADNc de la figure 1A peut être préparé ; PSTMG08 qui est le promoteur d'un gène endogène de tabac codant pour un ARNm d'où l'ADNc de la figure 1B peut être préparé ; PSTMG4B12 qui est le promoteur d'un gène endogène de tabac codant pour un ARNm d'où l'ADNc de la figure 2A peut être préparé ; PSTMG3C9 qui est le promoteur d'un gène endogène de tabac codant pour un ARNm d'où l'ADNc de la figure 2B peut être préparé.

5. ADN selon l'une quelconque des revendications 1 à 4, dans lequel ledit premier promoteur est un promoteur d'un gène correspondant à un clone d'ADNc pMON9608.

6. ADN selon l'une quelconque des revendications 1 à 5, dans lequel ledit ADN de stérilité femelle code pour une ribonucléase, en particulier l'ARNase T1.

7. ADN selon l'une quelconque des revendications 1 à 5, dans lequel ledit ADN de stérilité femelle code pour une BARNase.

8. ADN selon l'une quelconque des revendications 1 à 5, dans lequel ledit ADN de stérilité femelle code pour une ADNase, en particulier une endonucléase, particulièrement EcoRI ; une protéase, en particulier une papaine, particulièrement le zymogène de papaïne ou une protéine active de papaïne ; une glucanase ; une lipase, en particulier la phospholipase A₂ ; une peroxydase de lipide ; un inhibiteur de paroi cellulaire ; une toxine bactérienne, ou une ribozyme ; en particulier une ribozyme contre ARNm codé par l'un quelconque des gènes de type STMG, ou par un gène correspondant au clone ADNc pMON9608 ; ou en variante, dans lequel ledit ADN de stérilité femelle : code pour une ribonucléase, en particulier T₂ ou Rh ; code pour une glycoprotéine codée par les allèles S1, S2, S3, S6 ou S7, en particulier de Nicotiana alata ; ou est un ADN anti-sens, en particulier un ADN anti-sens codant pour un ARN qui est complémentaire à l'ARNm d'un gène de type STMG.

9. ADN selon l'une quelconque des revendications 1 à 5, dans lequel ledit ADN de stérilité femelle code pour une enzyme qui catalyse la synthèse d'une phytohormone, en particulier l'enzyme codée par le gène 4 de ADN-T d'Agrobacterium, ou une enzyme codée par le gène 1 et/ou le gène 2 d'ADN-T d'Agrobacterium.

10. ADN selon l'une quelconque des revendications 1 à 5, dans lequel ledit ADN de stérilité femelle code pour une polymérase d'ARN dépendante d'un virus, en particulier une TNV réplicase, sous la régulation dudit premier promoteur ; et un brin négatif d'un gène codant pour ledit premier polypeptide ou protéine ; ledit gène étant dans la même unité transcriptionnelle que, et sous la régulation de, un promoteur, tel qu'un autre premier promoteur, capable de diriger l'expression dudit brin négatif dans la cellule ; ledit gène étant également fusionné à son extrémité 3' à un promoteur subgénomique viral, tel que le promoteur TNV subgénomique, qui est spécifiquement reconnu par ladite polymérase d'ARN dépendante de virus.

11. ADN selon l'une quelconque des revendications 1 à 10, dans lequel ledit ADN comprend de plus :
(e) un premier ADN codant pour un peptide de transit capable de transporter ledit premier polypeptide ou protéine dans un chloroplaste ou une mitochondrie desdites cellules d'organe de reproduction femelle ; ledit premier ADN étant dans la même unité transcriptionnelle que ledit ADN de stérilité femelle et ledit premier promoteur, et situé entre ledit ADN de stérilité femelle et ledit premier promoteur.

12. ADN selon l'une quelconque des revendications 1 à 10, dans lequel ledit ADN comprend de plus :
(e) un troisième ADN codant pour un peptide signal sécrétoire capable de sécréter ledit premier poly-peptide ou protéine à l'extérieur de la cellule d'un organe de reproduction femelle d'une plante ; ledit troisième ADN étant dans la même unité transcriptionnelle que ledit ADN de stérilité femelle et ledit premier promoteur, et situé entre ledit ADN de stérilité femelle et ledit premier promoteur.

13. ADN selon l'une quelconque des revendications 1 à 12, qui comprend également un deuxième ADN chimérique, adjacent audit premier ADN chimérique, ledit deuxième ADN chimérique comprenant :
(c) un ADN marqueur codant pour un deuxième ARN, polypeptide ou protéine qui, quand il est présent au moins dans un tissu spécifique ou dans au moins des cellules spécifiques d'une plante, rend ladite plante facilement séparable des autres plantes qui ne contiennent pas ledit second ARN, polypeptide ou protéine dans ledit tissu spécifique ou lesdites cellules spécifiques ; et
(d) un deuxième promoteur capable de diriger l'expression dudit ADN marqueur au moins dans ledit tissu spécifique ou lesdites cellules spécifiques ; ledit ADN marqueur étant dans la même unité transcriptionnelle que ledit, et sous la régulation dudit, deuxième promoteur.

14. ADN selon la revendication 13, dans lequel ledit ADN marqueur code pour une protéine inhibant ou neutralisant l'action d'un herbicide.

15. ADN selon la revendication 14, dans lequel ledit ADN marqueur est un gène de résistance à un herbicide, en particulier un gène conférant une résistance à un inhibiteur de glutamine synthétase, tel que la phosphinothricine.

16. ADN selon la revendication 15, dans lequel ledit ADN marqueur est un gène sfr ou sfrv.

17. ADN selon la revendication 14, dans lequel ledit ADN marqueur est un gène codant pour une enzyme cible modifiée pour un herbicide ayant une affinité inférieure pour l'herbicide, en particulier une 5-énolpyruvylshikimate-3 phosphate synthase modifiée en tant que cible pour un glyphosate ou une glutamine synthétase modifiée en tant que cible pour un inhibiteur de glutamine synthétase tel que la phosphinothricine.

18. ADN selon la revendication 13, dans lequel ledit ADN marqueur est un gène codant pour une protéine ou un polypeptide conférant une couleur à au moins ledit tissu spécifique ou lesdites cellules spécifiques, en particulier le gène A1 ou le gène GUS ; un gène codant pour une protéine ou un polypeptide conférant une tolérance au stress à ladite plante, en particulier le gène codant pour la Mn-superoxyde dismutase ; ou un gène codant pour une protéine ou un polypeptide conférant une résistance aux maladies ou aux nuisibles, en particulier un gène codant pour une endotoxine de *Bacillus thuringiensis* qui confère une résistance aux insectes ou un gène codant pour un peptide bactéricide qui confère une résistance bactérienne.

19. ADN selon l'une quelconque des revendications 13 à 18, dans lequel ledit deuxième promoteur est : un promoteur constitutif, en particulier un promoteur PNOS ou un promoteur POCS ; un promoteur inductible par blessure, en particulier un promoteur TR1' ou TR2' ; un promoteur qui dirige l'expression d'un gène sélectivement dans un tissu de plante ayant une activité de photosynthèse, ou un promoteur qui dirige l'expression d'un gène sélectivement dans des cellules de feuille, des cellules de pétale ou des cellules de graine, en particulier des cellules d'enveloppe de graine.

20. ADN selon la revendication 19, dans lequel ledit deuxième promoteur est un promoteur 35S ou un promoteur SSU.

21. ADN selon l'une quelconque des revendications 13 à 20, dans lequel ledit ADN comprend de plus :
(h) un deuxième ADN codant pour un peptide de transit capable de transporter ledit deuxième polypeptide ou protéine dans un chloroplaste ou une mitochondrie d'au moins ledit tissu spécifique ou lesdites cellules spécifiques ; ledit deuxième ADN étant dans la même unité transcriptionnelle que ledit ADN marqueur et ledit deuxième promoteur, et situé entre ledit ADN marqueur et ledit deuxième promoteur.

22. ADN selon l'une quelconque des revendications 13 à 20, dans lequel ledit ADN comprend de plus :
(f) un quatrième ADN codant pour un peptide signal de sécrétion capable de sécréter ledit deuxième polypeptide ou protéine à l'extérieur d'au moins le tissu spécifique ou les cellules spécifiques ; ledit quatrième ADN étant dans la même unité transcriptionnelle que ledit ADN marqueur et ledit deuxième promoteur, et situé entre ledit ADN marqueur et ledit deuxième promoteur.

23. ADN qui comprend l'ADN-T de pMG100 de la figure 3, pMG101 de la figure 4, pMG102 de la figure 5, pMG103 de la figure 6, pMG104 de la figure 7, ou pMG105 de la figure 8.

24. ADN selon l'une quelconque des revendications 1 à 23 qui est un ADN nucléaire d'une cellule d'une plante ou d'une graine.

25. Vecteur qui contiennent l'ADN selon l'une quelconque des revendications 1 à 23.

26. Cellule d'une plante qui contient l'ADN selon l'une quelconque des revendications 1 à 24.

27. Cellule d'une plante qui contient, intégré de façon stable dans son ADN nucléaire, l'ADN selon l'une quelconque des revendications 1 à 23.

28. Cellule selon la revendication 26 ou la revendication 27 qui peut être régénérée dans une plante qui est stérile femelle.

29. Culture de cellule végétale contenant des cellules végétales selon l'une quelconque des revendications 26 à 28.

30. Plante qui contient l'ADN selon l'une quelconque des revendications 1 à 24.

31. Plante qui contient l'ADN selon l'une quelconque des revendications 1 à 24 dans la totalité de ses cellules.

32. Plante selon la revendication 30 ou 31 qui est stérile femelle.

33. Plante selon l'une quelconque des revendications 30 à 32 qui est une plante hybride.

34. Plante stérile femelle contenant un ADN étranger incorporé dans le génome nucléaire de ses cellules, dans laquelle ledit ADN étranger comprend :
(a) un ADN de stérilité femelle codant pour un premier ARN, protéine ou polypeptide, capable quand il est produit dans des cellules d'un organe de reproduction femelle de ladite plante, de perturber de façon significative le métabolisme, le fonctionnement et/ou le développement de l'organe de reproduction femelle ; et
(b) un premier promoteur capable de diriger l'expression de cet ADN sélectivement dans des cellules des organes de reproduction femelle de ladite plante, ledit ADN de stérilité femelle étant dans la même unité transcriptionnelle que ledit, et sous la régulation dudit, premier promoteur,
à condition que, si ledit premier promoteur est un promoteur capable de diriger l'expression dudit ADN de stérilité femelle sélectivement dans des gamètes femelles, ou dans des cellules provenant de gamètes femelles, le génome nucléaire de ladite plante est homozygote.

35. Plante selon l'une quelconque des revendications 30 à 34 qui est choisie dans le groupe formé de la luzerne et du tabac.

36. Graine végétale qui contient l'ADN selon l'une quelconque des revendications 1 à 24.

37. Fruit sans graine d'une plante qui contient l'ADN selon l'une quelconque des revendications 1 à 24.

38. Procédé pour produire une plante stérile femelle et un matériel de reproduction pour ladite plante, qui comprend :
a) l'introduction d'un ADN étranger selon l'une quelconque des revendications 1 à 23 dans le génome nucléaire d'une cellule végétale afin d'obtenir une cellule végétale transformée ;
b) la régénération de ladite plante stérile femelle à partir de ladite cellule végétale transformée et ; éventuellement,
c) l'obtention dudit matériel de reproduction à partir de ladite plante stérile femelle qui comprend ledit ADN étranger.

39. Procédé selon la revendication 20, dans lequel ledit matériel de reproduction est une graine, en particulier une graine hybride, et qui comprend de plus :
a) la fourniture d'une plante qui contient l'ADN selon l'une quelconque des revendications 1 à 23 incorporé dans l'ADN nucléaire de la totalité de ses cellules et qui est stérile femelle ;
b) la pollinisation croisée i) de ladite plante stérile femelle, et ii) d'une plante fertile femelle, et
c) la récupération de ladite graine de ladite plante fertile femelle.

40. Procédé selon la revendication 20, dans lequel ledit matériel de reproduction est une graine, en particulier une graine hybride, qui comprend les étapes consistant :
a) à fournir une plante qui contient l'ADN selon l'une quelconque des revendications 13 à 23, comprenant à la fois ledit deuxième promoteur et ledit ADN marqueur, incorporé dans l'ADN nucléaire de la totalité de ses cellules, et qui est stérile femelle ;
b) à réaliser une pollinisation croisée i) de ladite plante stérile femelle, et ii) une plante fertile femelle sans ledit ADN marqueur et/ou ledit deuxième promoteur, et
c) à récupérer la graine à partir de ladite plante fertile femelle,
dans lequel ledit procédé comprend éventuellement l'élimination des plantes fertiles femelles non souhaitables sur la base de l'absence d'expression de l'ADN marqueur dans lesdites plantes fertiles femelles.

41. Procédé pour produire une graine, qui comprend les étapes consistant :
(a) à fournir une plante qui contient l'ADN selon l'une quelconque des revendications 13 à 23 incorporé dans l'ADN nucléaire de la totalité de ses cellules, qui est stérile femelle, et dans lequel ledit ADN marqueur est un gène de résistance à un herbicide, en particulier un gène conférant une résistance à un inhibiteur de glutamine synthétase tel que le gène sfr ou sfrv, ou un gène codant pour une enzyme cible modifiée pour un herbicide ayant une affinité inférieure pour un herbicide, en particulier une 5-énolpyruvylshikimate-3 phosphate synthase modifiée en tant que cible pour un glyphosate ou une glutamine synthétase modifiée comme cible pour un inhibiteur de glutamine synthase tel que la phosphinothricine,
(b) à réaliser une pollinisation croisée i) de ladite plante stérile femelle, et ii) d'une plante fertile femelle sans ledit ADN marqueur et/ou ledit deuxième promoteur, et
(c) à récupérer la graine provenant de ladite plante fertile femelle,
dans lequel ledit procédé comprend l'élimination des plantes fertiles femelles non souhaitables par l'application dudit herbicide, en particulier un herbicide contenant de la phosphinothricine.

42. Procédé selon la revendication 41, dans lequel ladite graine est une graine hybride.

43. Procédé selon l'une quelconque des revendications 41 ou 42, dans lequel ladite plante stérile femelle contient en plus dudit gène de résistance à un herbicide au moins un autre ADN marqueur intégré de façon stable dans le génome nucléaire de ses cellules dans le même locus génétique que ledit ADN de stérilité femelle et ladite plante fertile femelle ne contient pas ledit autre ADN marqueur.

44. Procédé selon l'une quelconque des revendications 39 et 43, dans lequel la plante fertile femelle est stérile mâle.

45. Procédé selon la revendication 44, dans lequel ladite plante fertile femelle et stérile mâle comprend un ADN étranger incorporé dans le génome nucléaire de ses cellules, dans lequel ledit ADN étranger comprend :
(a) un ADN de stérilité mâle codant pour un troisième ARN, protéine ou polypeptide, capable quand il est produit dans des cellules d'étamine de ladite plante, de perturber significativement le métabolisme, le fonctionnement et/ou le développement des cellules d'étamine ;
(b) un troisième promoteur capable de diriger l'expression de cet ADN sélectivement dans des cellules d'étamine de ladite plante, ledit ADN de stérilité mâle étant dans la même unité transcriptionnelle que ledit, et sous la régulation dudit, troisième promoteur ;
à condition que, si ledit troisième promoteur est un promoteur capable de diriger l'expression dudit ADN de stérilité mâle sélectivement dans des cellules de pollen, le génome nucléaire de ladite cellules végétale transformée est homozygote.

46. Procédé selon la revendication 45, dans lequel ledit ADN étranger de ladite plante fertile femelle stérile mâle comprend également :
(d) un deuxième ADN marqueur codant pour un quatrième ARN, protéine ou polypeptide qui, quand il est présent au moins dans un tissu spécifique ou dans au moins des cellules spécifiques de ladite plante, rend ladite plante facilement séparable des autres plantes qui ne contiennent pas ledit quatrième ARN, protéine ou polypeptide dans ledit tissu spécifique ou lesdites cellules spécifiques ; et
(e) un quatrième promoteur capable de diriger l'expression dudit deuxième ADN marqueur au moins dans ledit tissu spécifique ou lesdites cellules spécifiques ; ledit deuxième ADN marqueur étant dans la même unité transcriptionnelle que ledit, et sous la régulation dudit, quatrième promoteur.

47. Procédé selon l'une quelconque des revendications 45 ou 46, dans lequel ledit ADN étranger comprend de plus :
(f) un cinquième ADN codant pour un peptide de transit afin de transporter ledit troisième polypeptide ou protéine dans un chloroplaste ou une mitochondrie desdites cellules d'étamine ; ledit cinquième ADN étant dans la même unité transcriptionnelle que ledit ADN de stérilité mâle et ledit troisième promoteur, et situé entre ledit ADN de stérilité mâle et ledit troisième promoteur ; et/ou
(g) un sixième ADN codant pour un peptide de transit capable de transporter ledit quatrième polypeptide ou protéine dans un chloroplaste ou une mitochondrie d'au moins ledit tissu spécifique ou lesdites cellules spécifiques ; ledit sixième ADN étant dans la même unité transcriptionnelle que ledit deuxième ADN marqueur et ledit quatrième promoteur, et situé entre ledit deuxième ADN marqueur et ledit quatrième promoteur.

48. Paire de plantes parents pour produire des graines comprenant : a) une plante parent stérile femelle contenant l'ADN selon l'une quelconque des revendications 1 à 23 incorporé dans l'ADN nucléaire de la totalité de ses cellules, et b) une plante parent fertile femelle.

49. Paire de la revendication 48, dans laquelle ladite plante parent stérile femelle et ladite plante parent fertile femelle appartiennent à différentes lignées.

50. Paire de la revendication 48, dans laquelle ladite plante parent stérile femelle et ladite plante parent fertile femelle proviennent de la même lignée consanguine.

51. Paire selon l'une quelconque des revendications 48 à 58, dans laquelle ladite plante parent fertile femelle est stérile mâle.
